# EUROPEAN PATENT SPECIFICATION

(11) **EP 4 230 189 B1**
(45) Date of publication and mention of the grant of the patent: **20.08.2025**
(21) Application number: 23157697.6
(22) Date of filing: 21.02.2023
(51) Int. Cl.: A61K 8/06, A61K 8/27, A61K 8/34, A61K 8/73, A61Q 19/00

(54) **EMULSIFYING COMPOSITION STABILIZED BY POWDERS**
DURCH PULVER STABILISIERTE EMULGIERENDE ZUSAMMENSETZUNG
COMPOSITION ÉMULSIFIANTE STABILISÉE PAR DES POUDRES

(30) Priority: 21.02.2022 IT 202200003131
(43) Date of publication of application: 23.08.2023
(73) Proprietor: Unifarco S.p.A., 32035 Santa Giustina (BL) (IT)
(72) Inventor: Baratto, Giovanni, 32035 Santa Giustina BL (IT); Semenzato, Alessandra, 35131 Padova PD (IT); Costantini, Alessia, 35131 Padova PD (IT); Tafuro, Giovanni, 35131 Padova PD (IT)
(74) Representative: Perani & Partners S.p.A.

(56) References cited:
- US-B1- 6 620 407
- US-B2- 6 558 683
- PANG BO ET AL: "Recent progress on Pickering emulsions stabilized by polysaccharides-based micro/nanoparticles", ADVANCES IN COLLOID AND INTERFACE SCIENCE, ELSEVIER, NL, vol. 296, 8 September 2021 (2021-09-08), XP086808877, ISSN: 0001-8686, [retrieved on 20210908], DOI: 10.1016/J.CIS.2021.102522
- TAFURO GIOVANNI ET AL: "Stability and Application Properties of Surfactant-Free Cosmetic Emulsions: An Instrumental Approach to Evaluate Their Potential", COSMETICS, vol. 9, no. 6, 18 November 2022 (2022-11-18), pages 123, XP093251175, ISSN: 2079-9284, Retrieved from the Internet <URL:https://www.mdpi.com/2079-9284/9/6/123/pdf> DOI: 10.3390/cosmetics9060123

## Description

### FIELD OF THE INVENTION

The field of the present invention relates to stable oil-in-water emulsions free of emulsifiers.

### BACKGROUND ART

Cosmetic products are "substances or preparations, other than medicinal products, intended to be applied on the external surfaces of the human body (...), or on the teeth and mucous membranes of the mouth with the aim of cleaning them, perfuming them, modifying their appearance, correcting their odours, protecting them or keeping them in good condition." Italian law no. 173 of 11 October 1986, Transposition of Directive 76/768/EEC.

Among all the technical forms marketed, emulsions are certainly the most complex and widely used: they are multi-phase systems in which the components, oily and aqueous, have simultaneous and synergistic action. The most widespread and preferred by the consumer are those of the oil-in-water type, since they are capable of combining sensory pleasantness with functionality and effectiveness: the components are released in a regulated manner on the skin, the lipid fraction is easily spreadable in a thin layer and finally the broad extension of the *interfacies* allows an effective activity of the active ingredients both superficially and in the deeper areas of the stratum corneum. However, the formulation of cosmetic emulsions is very complex, since it is necessary to reach the optimum state of stability and at the same time to respond to the functional requirements of effectiveness and pleasantness. D'Agostinis G., Mignini E., Manuale del cosmetologo: Ricerca applicata, Progettazione, Engineering, Produzione, Marketing, Packaging, Discipline collegate, Tecniche Nuove, Milan 2007, 346-355.

To improve the stability of an emulsion, several parameters can be acted on such as temperature, the size of the dispersed particles or droplets, the difference in polarity and density of the phases and the viscosity of the continuous or dispersing phase. However, these expedients alone are not sufficient and it is therefore necessary to add a third component, the emulsifier, which is capable of avoiding the aggregation of the dispersed particles, ensuring long-term stability of the product.

Many of the commercial formulations include surfactants as emulsifiers. Surfactants are amphiphilic molecules consisting of a hydrophobic tail and a polar head, by virtue of which they are capable of positioning themselves at the interface of the two phases of the emulsion. Exposing the head to the water phase and the hydrocarbon tail to the oil phase, they form a layer which coats the surface of the droplets and reduces tension at the interface; thereby the rate of potential energy not dissipated is lowered and the system is stabilized.

There are three different categories of surfactants: anionic, cationic, and non-ionic. Surfactants can be petroleum-based or of vegetable origin, and are found in cleaning products, detergents and various cosmetic products, such as shampoos, shower gels and bath products.

### Problem of the prior art

The disadvantage of using traditional surfactants is that they contribute to the depletion of a non-renewable resource and are highly polluting. Furthermore, they are poorly or not at all biodegradable and can release toxic chemicals when they decompose.

In addition, traditional surfactants are usually used in slight excess, so they can also be located outside the interface between immiscible, free liquids in the form of monomers or micelles. When these structures are small enough to penetrate the hair follicles, they can interact with the protein and lipid components present in the stratum corneum, denaturing them. The nature of the composition of the lipid cement is therefore altered and this favours the penetration of chemical or pathogenic components into the deeper layers of the skin, where the keratinocytes reside. In the presence of cell damage at the level of this cell type, real inflammatory responses are produced, which can lead to irritant contact dermatitis or allergic contact dermatitis, depending on whether the immune system is involved and therefore the production of antibodies. Therefore, surfactants, even those of plant origin, can cause skin irritations and allergies. Sewerin A., Interactions between surfactants and the skin - Theory and practice, Advances in Colloid and Interface Science, vol. 256, 2018, 242-255.

The use of powders to achieve the stabilization of emulsions for cosmetic use is, to date, a little explored field. These systems were not very successful due to the small variety of usable materials that limited the scope thereof.

US 6 620 407 B1 discloses oil-in-water emulsions comprising an oil phase, an aqueous phase, and a modified polysaccharide in solid form having both hydrophilic and lipophilic properties and which is dispersible in water and oil, and does not have thickening properties such as modified starch. ZnO particles can also be advantageously added. The preparation is free of surfactants.

### SUMMARY OF THE INVENTION

The applicant has now found that it is possible to stabilize an oil-in-water emulsion without having to resort to the use of surfactants, by virtue of the presence of a mixture of three distinct powders in specific concentrations with respect to the oil phase and in specific ratios to each other.

An object of the present invention is therefore an oil-in-water emulsion free of surfactants comprising:
(i) zinc oxide powder;
(ii) a powder mixture comprising or consisting of maize starch;
(iii) a powder comprising or consisting of a polysaccharide from bacterial fermentation;
(iv) an oil phase;
(v) water.

This emulsion is characterized in that:
(I) the total concentration of the zinc oxide powder (i) and the powder mixture comprising maize starch (ii) is comprised between 3 and 12% by weight on the total weight of said oil-in-water emulsion;
(II) the concentration of the total polymer is comprised between 0.05% and 0.7% by weight on the total weight of said oil-in-water emulsion;
(III) the concentration of the oil phase (iv) is comprised between 5 and 50% by weight on the total weight of said emulsion;
(IV) the weight ratio of (i)/(ii) is comprised between 1:5 and 5:1;
(V) (ii) is a maize starch mixture containing between 50 and 75% of said starch, between 25 and 50% by weight of polyvinyl alcohol and between 5 and 10% by weight of glycerine on the total weight of the mixture;
(VI) (iii) is an extract fermented from *Sphingomonas* and consists of mixtures of polysaccharides with average molecular weight comprised between 2.85 and 5.20 million Da at low charge density in which the repeating unit in the chain consists of 4 sugars, of which one is functionalized with a carboxylate group, while the side chain consists of repeating units with two neutral sugars.

A further object of the present invention are cosmetic formulations comprising or consisting of the oil-in-water emulsion according to the present invention.

### DESCRIPTION OF THE FIGURES

Figure 1 shows in a graph obtainable by performing a texture analysis using the texture Analyzer TMS-PRO, Food Technology Corporation, four of the five parameters which characterize texture: (Consistency, Firmness, Adhesiveness, Cohesiveness).
Figure 2 graphically shows the fifth parameter, Stringiness, obtained using the aforesaid device, texture Analyzer TMS-PRO, Food Technology Corporation.
Figure 3A shows the 40x magnification optical microscope photo of the oil-in-water emulsion of A7.5 O40 and figure 3B the photo with the same magnification of the oil-in-water emulsion of B7.5 O40.
Figure 4A shows the 40x magnification optical microscope of the oil-in-water emulsion AB10 O40; figure 4B shows an optical microscope photo of the same emulsion with 100x magnification.
Figure 5 shows the radar graph related to the texture parameters of binary systems, as the solid phase AB increases.
Figure 6 shows the radar graph related to the texture parameters of binary systems, reducing the oil phase O.
Figure 7 shows the flow curve as a function of the deformation rate of the hydrogels with Sphingomonas ferment extract polysaccharides obtained by fermentation from *Sphingomonas.*
Figure 8 depicts the curve of the trend of the modules G' and G" as a function of the oscillation amplitude for the hydrogels containing Sphingomonas ferment extract (polysaccharides obtained by fermentation from *Sphingomonas*).
Figure 9 shows the curve of the trend of the modules G' and G" as a function of the oscillation amplitude for the hydrogels containing Sphingomonas ferment extract (polysaccharides obtained by fermentation from *Sphingomonas*)
Figure 10 shows the texture curves from which the four parameters Consistency, Firmness, Adhesiveness, Cohesiveness of hydrogels with increasing concentration of C that is Sphingomonas ferment extract (polysaccharides obtained by fermentation from *Sphingomonas*) can be identified.
Figure 11 shows the graphs related to the 5 oil-in-water emulsion texture parameters of binary systems ACO40 (zinc oxide and polysaccharides obtained by fermentation from *Sphingomonas*) when varying the concentrations of zinc oxide in systems AC in which A is zinc oxide and C is Sphingomonas ferment extract (polysaccharides obtained by fermentation from *Sphingomonas.*
Figure 12 shows a 40x magnification light microscope photo of the binary emulsion B5C 040 where B is Zea mays starch, Polyvinyl alcohol, Glycerin).
Figure 13 is the radar graph related to the five texture parameters of binary systems BC as the concentration of B (mixture comprising maize starch) varies.
Figure 14 is the radar graph related to the five texture parameters of binary systems BC as the concentration of the oil component varies.
Figure 15 shows the trend of the modules G' and G" as a function of the oscillation amplitude for binary systems BC as the concentration of B varies.
Figure 16 shows the trend of the modules G' and G" as a function of the oscillation amplitude for binary systems BC as the concentration of the oil component varies.
Figure 17A shows the 40x magnification optical microscope photo of the ternary emulsion ABC0.1; figure 17B that of B) ABC0.3; figure 17C that of B) ABC0.3.
Figure 18 shows the trend of the modules G' and G" as a function of the oscillation amplitude for ternary systems ABCO40 as the concentration of *Sphingomonas* ferment extract C) increases compared to the values G' and G" of a binary system ABO40.
Figure 19 shows the trend of the modules G' and G" as a function of the oscillation frequency for ternary systems ABCO40 as C increases compared to the binary system ABO40.
Figure 20 shows the trend of the modules G' and G" as a function of the oscillation amplitude for ternary systems ABCO10 as the concentration of (C) increases compared to the values G' and G" of a binary system ABO10.
Figure 21 shows the trend of the modules G' and G" as a function of the oscillation frequency for ternary systems ABCO10 as C increases compared to the binary system ABO10.
Figure 22 shows the comparison histograms of the Firmness texture parameter of the ternary systems ABCO40 and ABCO10 as the concentration of the component C varies.
Figure 23 shows the comparison histograms of the Adhesiveness texture parameter of the ternary systems ABCO40 and ABCO10 as the concentration of component C varies.
Figure 24 shows the comparison histograms of the Stringiness texture parameter of the ternary systems ABCO40 and ABCO10 as the concentration of the component C varies.
Figure 25 shows the trend of the modules G' and G" as a function of the oscillation amplitude ABC as the concentration of the oil component varies.
Figure 26 shows the radar graph of the texture parameters of ternary systems ABC as the oil component varies.
Figure 27 shows the trend of the modules G' and G" as a function of the oscillation amplitude as the concentration of A+B varies.
Figure 28 shows the radar graph of ternary oil-in-water emulsions ABC as the concentration of A+B varies.

### DETAILED DESCRIPTION OF THE INVENTION

For the purposes of the present invention, "comprising" and "containing" do not exclude the presence of further components other than those mentioned after such a definition.

The definition "consisting of or consisting in" instead excludes the presence of further components not expressly mentioned after such a definition.

For the purposes of the present invention, binary systems are intended as oil-in-water emulsions containing only two types of solids.

The zinc oxide powder (i) is preferably less than 10 nm in size.

As far as the powder mixture comprising maize starch (ii) is concerned, for the purposes of the present invention a maize starch mixture (INCI name Zea mays starch) is intended containing between 50 and 75% of said starch, between 25 and 50% by weight of polyvinyl alcohol and between 5 and 10% by weight of glycerin on the total weight of the mixture.

This powder mixture preferably has an average particle size distribution D50 comprised between 5 and 15 µm.

As regards component (iii), it is an extract fermented from *Sphingomonas* and consists of mixtures of polysaccharides with average molecular weight comprised between 2.85 and 5.20 million Da at low charge density in which the repeating unit in the chain consists of 4 sugars, of which one is functionalized with a carboxylate group, while the side chain consists of repeating units with two neutral sugars.

Preferably the concentration of zinc oxide powder (i) + the total concentration of the powder comprising maize starch (ii) is comprised between 5 and 10% of the total weight of the oil-in-water emulsion.

Preferably the concentration of the polysaccharide obtained by bacterial fermentation (iii) is present in concentrations comprised between 0.1 and 0.5% by weight on the total weight of said aqueous emulsion.

Preferably the concentration of the oil phase (iv) is comprised between 10 and 40% by weight on the total weight of the oil-in-water emulsion object of the present invention.

Preferably the weight ratio of zinc oxide powder/powder mixture comprising maize starch is comprised between 1:2 and 2:1.

Even more preferably the weight ratio of zinc oxide powder/powder mixture comprising maize starch is 1:1.

Preferably the cosmetic formulations object of the present invention are for topical cutaneous use.

The object of the invention will be better illustrated by the following experimental part.

### Experimental study to obtain an emulsifying composition according to the invention

The objective of this study was to formulate a stable emulsified system of type O/W free of emulsifying surfactant, but stabilized only by powders which, positioning at the interface, act by forming a physical shell around the dispersed phase particles, so as to prevent the phase separation of the system. Currently most studies of emulsions without emulsifiers in the literature refer to emulsified systems and stable results on a small scale at room temperature, for a period of thirty days: an unreliable and unpredictable test, which does not ensure compliance with the essential stability requirements for a cosmetic product which must be placed on the market. In order to reach a technical form with potential application in the development of cosmetic products, it was thought to target the focus of the project on the formulation of systems capable of withstanding more intense stresses, such as those of the mechanical stability test in a centrifuge and the accelerated ageing test in a thermostated oven. Rheology and texture analyses also made it possible to characterize the physical-mechanical properties of the systems obtained and to evaluate the structural properties thereof.

The experimental study was divided into four steps 1-4:
1. First, an initial screening was carried out: two emulsions with different solid and oil phase content were set up, with the aim of evaluating the emulsifying capacity of the raw materials selected;
2. Subsequently, the behaviour of the raw materials in emulsion was studied, which allowed to obtain stable systems at room temperature in the 24 hours following the formulation, considering the powders or inorganic/organic raw materials taken individually. Therefore, the behaviour of the system was investigated when the concentration between the solid and oil phases varied, seeking, in addition to stability at room temperature, also mechanical stability (3000 rpm centrifuge);
3. Given that the emulsions stabilized by single powder did not lead to the desired results, three types of systems were set up in binary associations and their mechanical stability was investigated:
   - Systems with Zinc oxide and Zea mays starch, Polyvinyl alcohol, Glycerin ("binary system AB");
   - Systems with Zinc oxide and Sphingomonas fermented extract ("binary system AC");
   - Systems with (Zea mays starch, Polyvinyl alcohol, Glycerin) and *Sphingomonas* fermented extract ("binary system BC");
4. In order to reach the formulation of a stable emulsion which was at the same time fluid and sensorially pleasing, ternary systems were set up stabilized by Zinc oxide (Zea mays starch, Polyvinyl alcohol, Glycerin) and Sphingomonas fermented extract in association ("ternary system ABC"). The behaviour of the system was investigated to vary the concentration of solid phase, oil phase and also the ratio of Zinc oxide A and (Zea mays starch, Polyvinyl alcohol, Glycerin) B.

These systems were subjected to the centrifuge stability test, and characterized from a physical-mechanical perspective with rheology and texture analyses.

### 1. Raw material

### Organic materials:

### Cellulose 1 and Cellulose 2, INCI name: Cellulose;

Cellulose 1 and Cellulose 2 are two raw materials for cosmetic use with the INCI name "Cellulose" and are formed by micrometric particles of different shape: in the first case (cellulose 1) they are particles with a fibrous structure and high porosity of 30 µm, while in cellulose 2 the particles are spheroidal in shape in the size range between 7 µm and 12 µm. The choice to use raw materials which are qualitatively identical but with a different particle shape was made in order to identify any differences in emulsifying capacity dictated by the amount of surface area available for adsorption to the interface.

Component B; INCI name: Zea mays starch, Polyvinyl alcohol, Glycerin. is a maize starch modified with polyvinyl alcohol, with INCI name "Zea mays starch, Polyvinyl alcohol, Glycerin" with particle size in the range 5 µm-15 µm.
- Modified rice starch with INCI name "Distarch Phosphate" with 8 µm particles. The choice of using different starches subjected to modifications of different nature was made in order to investigate how such differences can modify their potential use as an emulsifier.

### Inorganic materials:

- INCI name: Silica, Cetyl alcohol; it is a silica modified with cetyl alcohol, thus rendered more lipophilic with respect to native silica, with INCI name "Silica, Cetyl alcohol". With a particle size of 7 µm and a balanced lipophilic and hydrophilic character, it is a good candidate for interface stabilization.
- Component A ZINC OXIDE (INCI name: Zinc oxide (physical sunscreen); its characteristic insolubility in water and oil makes it a good candidate for interface stabilization.

*Rheological modifiers:* Component C; INCI name: Sphingomonas fermented extract is a polysaccharide obtained from bacterial fermentation with the INCI name *"Sphingomonas* fermented extract". It has a viscous and suspending capacity and can act synergistically with other polymers of natural origin to emulsify and stabilize the system.

*Humectants*: GLYCERIN, INCI name: Glycerin.

*Emollients* (oil phase): INCI: Dicaprylyl carbonate; INCI: Caprylic/ Capric triglyceride.

*Salts*: INCI: Sodium Chloride.

### 2. Analytical methodologies

The prepared samples were subjected to different instrumental analyses to verify the stability of the emulsion over time, the physical-mechanical properties and the flow behaviour.

### 2.1 Preliminary stability test at room temperature

For the next 24 hours after preparation, each sample was subjected to a preliminary stability test at room temperature. After emulsification, the samples were left to rest in the container and were monitored to verify, from a macroscopic perspective, the presence of physical instability phenomena, such as the formation of creaming, sediment or the emergence of the oil phase. The samples that were stable in the preliminary stability test were then subjected to the mechanical stability test in a centrifuge.

### 2.2 Centrifuge mechanical stability test

Centrifugal testing is the most widely used method to evaluate mechanical stability over time; it involves the application of strong mechanical stress to the samples, centrifugal force, in order to accelerate any instability processes taking place in the emulsion. It is thereby possible to highlight instability phenomena, such as creaming, sedimentation and phase separation.

Centrifuge stability tests were conducted on samples allowed to rest for at least 24 hours from preparation; after this time, an exact amount of 5 grams was weighed into a single-use Falcon plastic tube for each sample. The test was conducted using MED INSTRUMENTS MPW-56 centrifuge, initially setting a speed of 3000 rpm for a time of 30 minutes. The samples which did not report instability phenomena after this first test were subjected to a second test at 4800 rpm, always for a time of 30 minutes.

### 2.3 Morphological analysis

Morphological analyses were performed with the LEICA DM1000 optical microscope, with 40x and 100x lenses and connected to LEICA LAS IMAGE image processing and storage software. The samples were prepared by placing a minimum amount on a standard slide, so as to create a homogeneous film that allowed to view a single image plane.

Optical microscope analyses allow to observe the morphology and structural organization of the sample. Specifically, it was possible to observe the presence of the different components of the emulsion, the size and morphology of the dispersed phase droplets, and instability or inhomogeneity phenomena.

### 2.4 Rheological analyses

Rheological analyses were conducted so as to obtain information about the internal structure and flow behaviour of the samples. The analyses were performed with a RHEOPLUS ANTON PAAR MCR 302 rheometer, with knurled plate-plate type plate geometry (PP50/P2), fixed gap at 1 mm and static temperature at 23 ± 0.05°C.

The analyses were performed two days after the sample preparation date. Each sample was subjected to continuous and oscillatory analysis. The data were acquired on a logarithmic scale and will be reported, commented and evaluated in section 4. Results and discussion.

### 2.4.1 Continuous motion analysis

*Controlled shear rate (CSR)*: the analysis makes it possible to obtain a flow curve which represents, as the rate of deformation varies between 0.001 s⁻¹ and 1000 s⁻¹, the variation in viscosity (η). It is a destructive analysis, at the end of which the sample presents irreversible structural changes, adapted to study the flow features of the product, simulating the application on the skin.

### 2.4.2 Oscillatory motion analyses

Oscillatory motion analyses allow to investigate the internal structure of the product; the system responses are represented by graphs that correlate the trend of the elastic and viscous components of the system (the modules) to the variation in amplitude and frequency of oscillation.

*Amplitude Sweep (AS)*: analysis carried out at a constant frequency (1Hz) and applying a variable strain gradient, in a defined range (0.01-1000%). This makes it possible to determine the linear viscoelasticity region (LVER), where the sample is not reversibly modified. Depending on the frequency set, the AS allows to determine the viscoelastic character of the system through the trend assumed by the modules G' (elastic module) and G" (viscous module): when G'>G'', solid-like behaviour is referred to, while when G''>G' the behaviour is defined as liquid-like.

*Frequency Sweep (FS)*: analysis carried out by keeping the applied stress constant and varying the frequency in a set range of values (10- 0.1 Hz). The deformation value chosen is comprised included in the linear viscoelasticity range, whereby the analysis does not irreversibly change the structure of the product. In particular, if in the investigated frequency range G'>G'' with a trend in the first two decades, it is a weak-gel structure stabilized by physical interactions or by chemical interactions of weak intensity; if the modules have the same trend, but are located between the third and the fourth decade, it is a strong-gel structure characterized by the presence of very tenacious chemical bonds. The structural difference is related to differences in spreadability during application, which is better for systems with weak interactions.

### 2.5 Texture Analysis

Texture analyses were carried out with Food Technology corporation's TMS-PRO TEXTURE ANALYZER, equipped with a 10 Newton load cell and a 2-centimetre diameter spherical nylon probe. The rate of analysis was set at 80 mm/min and the displacement, i.e. the penetration depth of the probe into the sample, at 10 mm.

The samples were analysed two days after preparation, more precisely the day after transfer into specific containers for analysis. These are 50 ml spherical jars, with a height of 5 cm and a diameter of 5.5 cm: these structural specifications allow the elimination of the edge effect that could alter the analysis data.

Each analysis was repeated three times on each sample, waiting five minutes between one analysis and the next to allow structural recovery; for the most consistent samples, before each analysis the surface was levelled with a spatula.

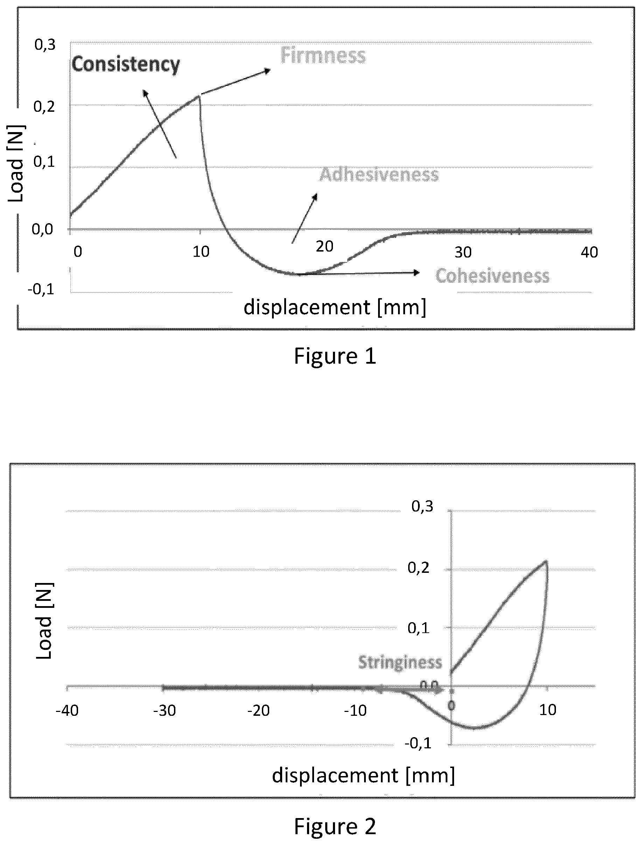

The tool allows to obtain curves in a graph of *Load (N)* vs *Displacement* (mm) as depicted in Figure 1 in which the following parameters can be identified:
- *FIRMNESS*: maximum peak of the positive curve [N];
- *CONSISTENCY*: area under the positive curve [N.mm];
- *ADHESIVENESS*: area under the negative curve [N.mm];
- *COHESIVENESS*: minimum peak of the curve [N];
- *STRINGINESS* not indicated in figure 1 represents the length of the filament [mm] formed when the probe re-emerges from the sample and the curve gradually returns to zero. This parameter can be more accurately obtained from the curve with non-cumulative displacement: it is represented by the distance between the zero point and the endpoint as shown in Figure 2

Conventionally, the numerical values obtained from texture analyses are the result of the average of the three measurements performed on the sample.

### 3. Formulation methods

The solid phase components were first used singly and then in association for making emulsions. The set up of the emulsions required the use of beakers of different capacities (50 mL, 100 mL, 150 mL and 250 mL), technical scale, analytical scale, heating plate with magnetic stirrer and Silverson L5T laboratory turboemulsifier.

The powders under study were used for the production of O/W emulsions. It was necessary to adopt different dispersion methods based on the chemical nature and properties of the raw materials considered, as well as the speed and stirring time. All the prototypes were formulated at a temperature of 40-45°C. All the concentrations will be reported in weight/weight percentages (%w/w).

### 3.1 Set-up of the emulsifying compositions with the raw materials for screening their emulsifying capacity

For the preliminary screening two different emulsions were prepared, varying the percentage concentration of powders and emollients: formulation 1 is characterized by 1% solid phase and 10% oil phase, while formulation 2 is characterized by 10% solid phase and 40% oil phase B. Both phases were brought to the temperature of 45°C and, depending on the organic or inorganic nature, the powder was dispersed, under stirring, in water (Table 1) or in the oil mixture or oil phase B (Table 2) until homogeneous.

**Table 1- formulation scheme of emulsions stabilized by organic powders**

| | | **1** (% p/p) | **2** (% p/p) |
|---|---|---|---|
| **PHASE A** | WATER | qs.100 | qs.100 |
| | GLYCERIN | 4 | 4 |
| | ORGANIC POWDERS | 1 | 10 |
| **PHASE B** | Dicaprylyl carbonate | 5 | 20 |
| | Caprylic/ Capric triglyceride | 5 | 20 |

**Table 2 formulation scheme of emulsions stabilized by inorganic powders**

| | | **1** (% p/p) | 2 (% p/p) |
|---|---|---|---|
| **PHASE A** | WATER | qs.100 | qs.100 |
| | GLYCERIN | 4 | 4 |
| **PHASE B** | INORGANIC POWDERS | 1 | 10 |
| | Dicaprylyl carbonate | 5 | 20 |
| | Caprylic/ Capric triglyceride | 5 | 20 |

After the dispersion, the emulsification was carried out: phase B was added to phase A, under continuous stirring with Silverson L5T at 4500 rpm/min for about 5 minutes.

The preliminary screening of the *Sphingomonas* ferment extract was carried out in different manners with respect to the previous ones as shown in Table 3 below

**Table 3: formulation scheme of emulsions stabilized by Sphingomonas ferment extract**

| | | **1** (% p/p) | **2** (% p/p) |
|---|---|---|---|
| **PHASE A** | WATER | qs.100 | qs.100 |
| | SODIUM CHLORIDE | 0.05 - 0.15 | 0.05 - 0.15 |
| **PHASE B** | GLYCERIN | 4 | 4 |
| | *Sphyngomonas* ferment extract | 0.1 - 0.3 | 0.1 - 0.3 |
| **PHASE C** | Dicaprylyl carbonate | 5 | 20 |
| | Caprylic/ Capric triglyceride | 5 | 20 |

Sodium chloride in a 1:2 ratio with respect to the amount of gum was added to the water phase heated to 45°C. In a separate beaker, the *Sphingomonas* ferment extract was moistened with glycerin, and later joined to phase A after salt solubilization. Once the dispersion occurred, when the two phases reached the same temperature, the emulsification was carried out, inserting phase C under continuous stirring with Silverson L5T at 4500 rpm/min for about 5 minutes.

For the sake of simplicity, from this point on, the oil mixture will be referred to more generically as the total "oil phase", since the nature of its components and their ratio (1:1), were kept unchanged throughout the study.

### 3.2 Set-up of systems emulsified by ZINC OXIDE and (Zea mays starch, Polyvinyl alcohol, Glycerin)

To investigate the emulsifying capacities of ZINC OXIDE and (Zea mays starch, Polyvinyl alcohol, Glycerin), the behaviour of the system was studied as the solid and oil phase concentration varied (Table 4)

**Table 4: general formulation scheme of emulsions stabilized by single powder.**

| | | **1** (% p/p) | **2** (% p/p) |
|---|---|---|---|
| **PHASE A** | WATER | qs.100 | qs.100 |
| | GLYCERIN | 4 | 4 |
| | (Zea mays starch, Polyvinyl alcohol, Glycerin) | 5-7.5-10-15 | / |
| **PHASE B** | ZINC OXIDE | / | 5-7.5-10-15 |
| | TOTAL OIL PHASE | 20-30-40 | 20-30-40 |

Depending on the nature, the powder was dispersed, under stirring and at the temperature of 45°C, in the phase closest thereto. Upon homogeneous dispersion, the emulsification was carried out: phase B was added to phase A, under continuous stirring, and with Silverson L5T at 4500 rpm/min for about 5 minutes.

### 3.3 Set-up of systems emulsified by powder associations

### 3.3.1 Binary systems

To study the behaviour of emulsions stabilized by binary powder associations, three different types of formulations were set up, as indicated in Table 5; formulation 1 contains ZINC OXIDE and (Zea mays starch, Polyvinyl alcohol, Glycerin) (system AB), formulation 2 (Zea mays starch, Polyvinyl alcohol, Glycerin) and *Sphingomonas ferment extract* (system BC) and finally formulation 3 ZINC OXIDE and *Sphingomonas ferment extract* (system AC).

**Table 5: formulation skeleton of emulsions stabilized by binary powder association.**

| | | **1** (% p/p) | **2** (% p/p) | **3** (% p/p) |
|---|---|---|---|---|
| **PHASE A** | WATER | qs.100 | qs.100 | qs.100 |
| | SODIUM CHLORIDE | / | 0.05 | 0.05 |
| | (Zea mays starch, Polyvinyl alcohol, Glycerin) | 2.5-5-7.5 | 5-10 | / |
| **PHASE B** | GLYCERIN | / | 4 | 4 |
| | *Sphyngomonas* ferment extract | / | 0.1 | 0.1 |
| **PHASE C** | ZINC OXIDE | 2.5-5-7.5 | / | 5-10 |
| | TOTAL OIL PHASE | 40-30-20 | 40-10 | 40-10 |

In formulation 1 the powders are present in a ratio of 1:1. Each was dispersed, under stirring and at the temperature of 45°C, in the phase closest thereto. Upon complete dispersion the emulsification was carried out: phase C was added to phase A, under continuous stirring and with Silverson L5T at 4500 rpm/min for about 5 minutes.

Instead in formulations 2 and 3, phase A was prepared, under stirring at 45°C, solubilizing the salt in water, in a 1:2 ratio with respect to the amount of gum. The powder considered was dispersed, always under stirring and at the same temperature, in the phase closest thereto. Upon complete and homogeneous dispersion, the *Sphingomonas* ferment extract previously moistened with glycerin was added to phase A. The phases are kapt under stirring until homogeneus, then the emulsification is carried out: phase C was added to phase A, under continuous stirring and with Silverson L5T at 4500 rpm/min for about 5 minutes.

### 3.3.2 Ternary systems

To study the behaviour of emulsions stabilized by ternary powder associations, formulations containing ZINC OXIDE, (Zea mays starch, Polyvinyl alcohol, Glycerin) and *Sphingomonas* ferment extract (systems ABC) were set up, as reported in Table 6

**Table 6: formulation skeleton of ternary emulsions stabilized by two powders in 1:1 ratio and by Sphingomonas ferment extract.**

| | | **1** (% p/p) |
|---|---|---|
| **PHASE A** | WATER | qs.100 |
| | SODIUM CHLORIDE | 0.05-0.15-0.25 |
| | (Zea mays starch, Polyvinyl alcohol, Glycerin) | 5-3.75-2.5 |
| **PHASE B** | GLYCERIN | 4 |
| | *Sphyngomonas* ferment extract | 0.1-0.3-0.5 |
| **PHASE C** | ZINC OXIDE | 5-3.75-2.5 |
| | TOTAL OIL PHASE | 40-30-20-10 |

Phase A was prepared under stirring at 45°C, first solubilizing sodium chloride (in a 1:2 ratio with respect to the amount of gum). Subsequently the starch is dispersed. Upon complete and homogeneous dispersion, the *Sphingomonas* ferment extract previously moistened with glycerin was added to phase A. Meanwhile, phase C is prepared, suspending ZINC OXIDE in the oil phase under stirring at the temperature of 45°C. Homogeneity was reached for both phases, then proceeding with emulsification: phase C was added to phase A, under continuous stirring and with Silverson L5T at 4500 rpm/min for about 5 minutes.

### 4. Results and discussion

The study was carried out with the aim of formulating an emulsified system of O/W type free of surfactant and stabilized only by solid components, whose particles are capable of adsorbing to the interface physically protecting the dispersed phase droplets. The project was articulated in several steps, so as to obtain a prototype characterized by stability over time and sensory pleasantness in all application phases, from pick-up to the after-feel.

The stability at room temperature was initially assessed for each sample, followed by centrifuge testing to simulate a condition of high mechanical stress. Observation under optical microscope allowed to detect the morphological features of the prepared systems, also highlighting the presence of instability phenomena; it was thereby possible to obtain satisfactory indications on the stability profile of the samples observed.

The most interesting samples with a suitable stability profile were subsequently characterized from the physical-mechanical perspective through Texture Analysis and rheological analyses, conducted in continuous and oscillatory motion. These were useful for obtaining information on the internal structure of the sample, to study the flowability and finally to identify which structural components determine the final application and sensory properties of the system.

### 4.1. Powder emulsifying ability screening

The first part of the work focused on identifying some recurring categories of substances to obtain a formulation free of emulsifiers, preferably for cosmetic use. Subsequently, the individual raw materials were chosen by virtue of the features necessary for positioning at the interface.

In order to obtain preliminary information on the affinity for the oil and water phases used in the formulation of emulsions, the raw materials presented were subjected to a preliminary test to assess the oil-absorbing capacity thereof. Using a mixture of (*Triethylexanoin*) and (*Dicaprylyl carbonate*) in a 1:1 ratio and weighing one gram of substance, the amount of oil necessary for the formation of a homogeneous paste, which does not ooze oil, is calculated; the values are then calculated on an amount of powder equal to 100g and the value of the wettability index was calculated as the ratio of powder mass (g)/oil mass (Table 7).

**Table 7: data obtained from the oil mixture absorption tests.**

| **INCI** | **OBTAINED ABSORPTION (per 100 g of substance)** | **WETTABILITY INDEX (100 g substance/ g oil mixture)** |
|---|---|---|
| *Cellulose* | 237g | 0.42 |
| *Cellulose* | 272g | 0.37 |
| *Zinc Oxide* | 162g | 0.62 |
| *Zea Mays Starch, Polyvinyl Alcohol, Glycerin* | 132g | 0.76 |
| *Silica, Cetyl alcohol* | 90g | 1.11 |
| *Distarch Phosphate* | 98g | 1.02 |
| *Sphingomonas fermented extract* | 104g | 0.96 |

The same were then used to produce two different emulsions, different for the percentage concentration of solid phase and oil phase: in the first case the solid phase at 1% and the oil phase at 10%, while in the second both increased respectively to 10% and 40%. Only *Sphingomonas* ferment extract, considering the indications given in the technical data sheet, was used differently: in both emulsions containing 10% and 40% of the oil phase, it was inserted according to two different concentrations: 0.1% and 0.3%.

The data regarding the emulsifying capacity of the individual raw materials in both formulations are reported in Table 8

**Table-8 - screening data on the emulsifying capacity of the selected raw materials.**

| INCI Name | % solid phase (p/p) | % oil phase (p/p) | Emulsifying capacity | Stability 24 h, ambient T |
|---|---|---|---|---|
| *Cellulose 1* | 1 | 10 | YES | SEPARATES |
| | 10 | 40 | NO | // |
| *Cellulose 2* | 1 | 10 | YES | SEPARATES |
| | 10 | 40 | NO | // |
| ***Zinc Oxide*** | 1 | 10 | YES | // |
| | 10 | 40 | YES | **STABLE** |
| ***Zea Mays Starch, Polyvinyl Alcohol, Glycerin*** | 1 | 10 | YES | **STABLE** |
| | 10 | 40 | YES | **STABLE** |
| *Silica, Cetyl alcohol* | 1 | 10 | NO | // |
| | 10 | 40 | NO | // |
| *Distarch Phosphate* | 1 | 10 | YES | // |
| | 10 | 40 | YES | // |
| *Sphingomonas fermented extract* | 0.1 | 10 | NO | // |
| | 0.1 | 40 | NO | // |
| | 0.3 | 10 | NO | // |
| | 0.3 | 40 | NO | // |

The only raw materials capable of emulsifying and forming stable systems one day after preparation were ZINC OXIDE and Zea mays starch, Polyvinyl alcohol, Glycerin. This can be explained considering the behaviour assumed by the raw materials during the oil absorption test.

In fact, ZINC OXIDE and Zea mays starch, Polyvinyl alcohol, Glycerin have intermediate oil absorption (and wettability index) values with respect to the other raw materials. The assumption of an intermediate behaviour confirms the experimental evidence that raw materials with partial affinity for both phases, oil and aqueous, are able to better stabilize the emulsion by means of the mechanism at the interface. On the contrary, raw materials with higher or lower oil absorption values denote a preponderant affinity (and thus a greater inclination to complete distribution) for one of the two phases, with the result that the system fails to be emulsified or after little time from phase separation.

These two raw materials, promising with respect to all the others, are carried forward in the next phase of the work with the aim of studying the features and stability of the emulsified systems formed in more depth.

### 4.2. Systems emulsified by ZINC OXIDE and Zea mays starch, Polyvinyl alcohol, Glycerin

In this second section, the stability at room temperature and in a centrifuge of emulsions stabilized by ZINC OXIDE (A5, A7,5, A10) and emulsions stabilized by Zea mays starch, Polyvinyl alcohol, Glycerin (B5, B7,5, B10) will be analysed, varying one or more parameters.

Initially, the behaviour of the system was studied as the solid phase and the oil phase (O) varied, however keeping their ratio constant at 1:4 (Table 9).

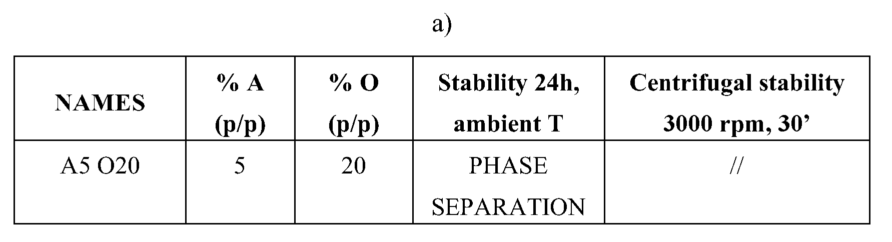

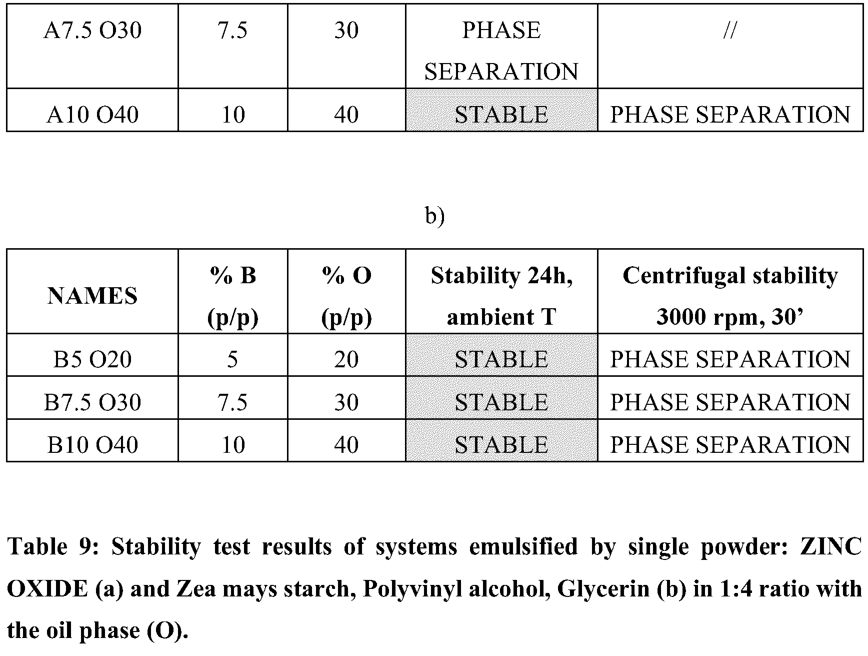

While the emulsions containing the starch are all stable at room temperature, among the emulsions with the zinc oxide, A10 O40, containing high percentages of both powder and oil, is stable. In fact, the decrease of the oil phase involves the increase of the water aliquot present in the system, which reduces the amount of interface available to the zinc: the separation occurs after a few minutes from the end of the emulsification in the Silverson.

In order to obtain systems stable in the centrifuge test conducted at 3000 rpm for 30 minutes, the behaviour of the emulsions was studied by keeping the oil phase (O) at 40% and varying the solid phase concentration (Table 10).

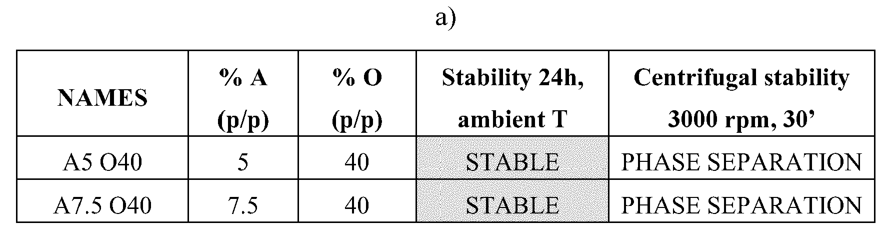

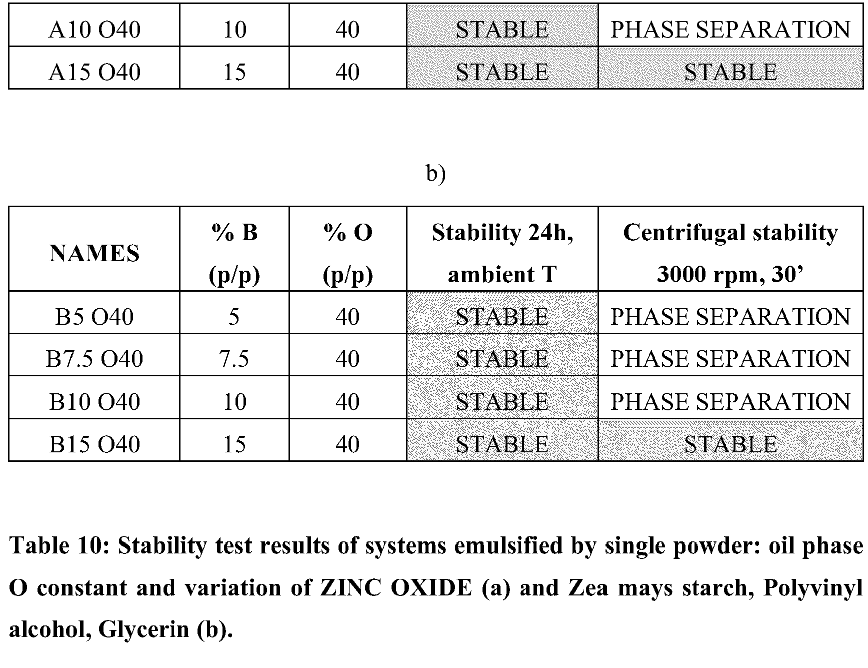

By keeping the oil phase fixed at 40%, stability at room temperature could be achieved for all the formulations, while centrifuge stability was only achieved for the formulations A15 O40 and B15 O40. In the case of formulations containing zinc oxide, the instability is represented by the emergence of the oil phase on the surface, while for maize starch there is the formation of a separate system in three phases: the surface and bottom phase remains emulsified, while in the central phase the liquid component is visible.

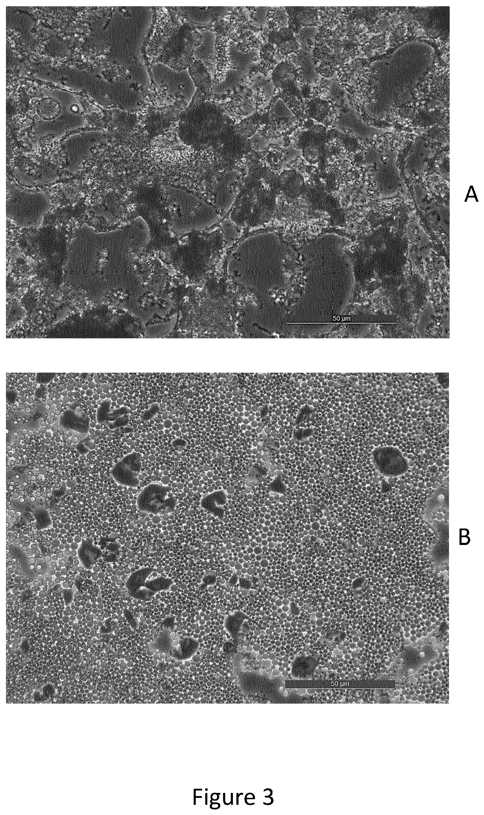

In order to investigate the system morphology, some emulsions were observed under light microscopy. As soon as it was subjected to the pressure of the glass cover, the emulsion A7.5 O40 broke: the oil escaped, leaving only a deposit of zinc oxide powder on the slide. Consistent with the results of the centrifuge test, it is a physically very unstable system that cannot retain oil, in fact in Figure 3A, the dispersed phase is absent and it is possible to see only the zinc which, once exposed to light, acquires a brownish-red colour. On the contrary, in the emulsion B7.5 O40, it is possible to distinguish all the components of the system: at first sight starch granules and a dense set of dispersed phase droplets are noticeable. These, in particular, turn out to be on average large and inhomogeneous and the size distribution is very wide. This data is also in agreement with the outcome of the centrifuge test, because systems with such morphology are more inclined to encounter instability phenomena which lead to the phase separation of the system (figure 3B).

This preliminary study allowed to establish that stable emulsions are formed only in the presence of high percentages of solid phase and oil phase, respectively at 15% and 40% w/w. However, these systems are characterized by a sensory profile which is hardly acceptable by the consumer, characterized by a pronounced sticky and oily after-feel due to the high oil phase concentration. Bearing in mind that the sensory aspect is an essential requirement for the formulation of a cosmetic, in subsequent sections systems containing associations of powders were worked with, so as to reconcile stability and sensoriality.

### 4.3. Systems emulsified by binary powder associations

The third part of the work focuses on the production of O/W systems emulsified by binary powder associations, containing ZINC OXIDE and Zea mays starch, Polyvinyl alcohol, Glycerin, with the aim of obtaining emulsions with more balanced and pleasant sensory features, and stabilized in a synergistic manner at the *interfacies* level.

### 4.3.1 SYSTEMS AB

Considering the raw materials with the best emulsifying capacities, binary systems were set up containing associations of ZINC OXIDE (A) and Zea mays starch, Polyvinyl alcohol, Glycerin (B) in constant ratio 1:1, which constitute the total solid phase present in the system (AB).

Initially, the behaviour of the system was studied by keeping the oil phase constant at 40% (O), and varying the percentage concentration of solid phase AB (Table 11).

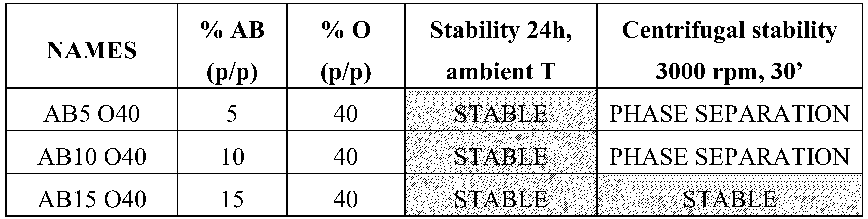

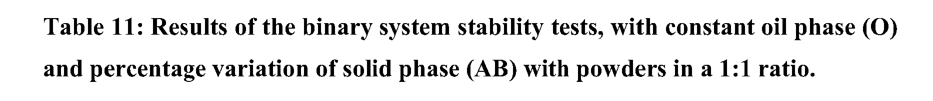

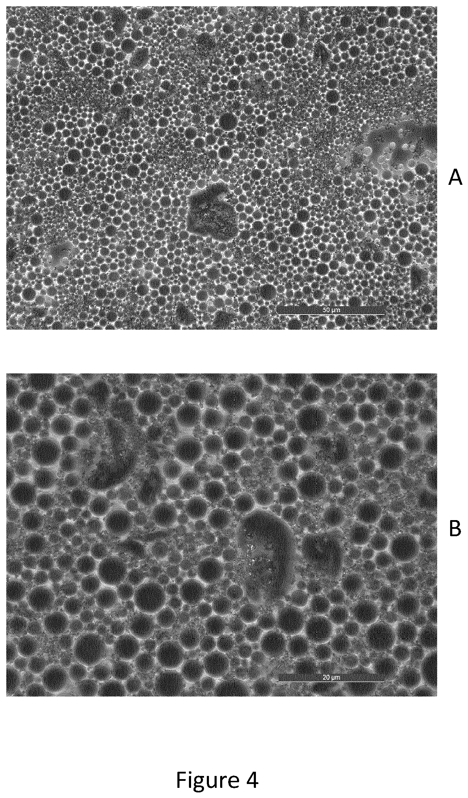

Similar to the systems emulsified by single powder, the only emulsion that was stable to the centrifuge test is AB15 O40, with high percentage concentrations of solid and oil phase, which has the same sensoriality problems explained in the previous section. The instability of the other samples was then also confirmed by electron microscope acquisitions: the emulsion AB 10 O40 demonstrates that it possesses, similarly to the systems emulsified by single powder, medium large dispersed phase droplets with a large dimensional heterogeneity (Figure A and Figure 4B).

Subsequently, the behaviour was studied by keeping the percentage of solid phase AB constant (10%) and reducing the oil phase O (Table 12).

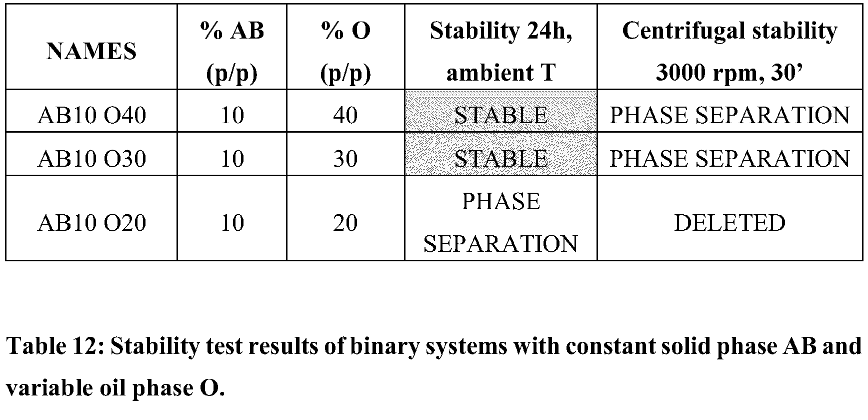

Therefore, decreasing the oil phase leads to a deterioration in stability, even at room temperature. Probably, the increase of the percentage of water phase, consequent to the decrease of the percentage of oils, leads to a massive reduction of the interface area available to the particles, as well as a decrease of the system viscosity, accelerating the onset of instability phenomena.

Despite the low stability profile, the samples were subjected to texture analyses to have some information on the structural and mechanical properties conferred by the association of these powders.

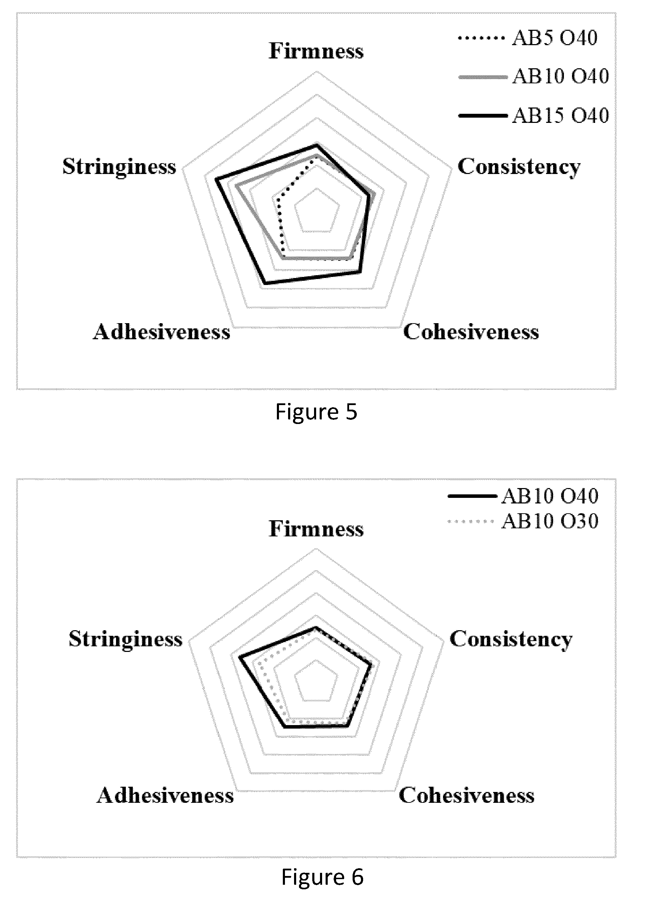

With the Texture Analysis it was possible to note how the increase in solid phase leads to an increase in all the texture parameters (Figure 5), but above all in Adhesiveness, Cohesiveness and Stringiness. The increase in these parameters affects the spreadability of the system which, being more structured, is more difficult due to the greater resistance exerted during the deformation; as the concentration of powder increases, the predisposition of the sample to remain attached to the probe increases, which therefore forms a longer filament, typical of sticky formulations.

By decreasing the oil phase, on the other hand, there is only a decrease in the Stringiness: the increase in free water results in the formation of more fluid systems characterized by a more difficult pick up (Figure 6).

Since it was therefore not possible to achieve system stability using only ZINC OXIDE and Zea mays starch, Polyvinyl alcohol, Glycerin, it was thought to reintroduce a raw material used in the initial screening but abandoned due to the absence of emulsifying capacity: *Sphingomonas* ferment extract. In fact, it acts by stabilizing the system since it viscosifies the water phase at low concentrations, manages to homogenize the dispersion of the components by virtue of the suspending properties and, being a rheological modifier, is capable of modifying the flow and sensory properties of the emulsion.

### 4.3.2 Characterization of Sphingomonas ferment extract

*Sphingomonas* ferment extract and its structural features were preliminarily studied by setting up hydrogels, dispersing the gum (C) in water in increasing concentrations (0.25%-0.5%-0.75%-1%). The samples were characterized by continuous motion and oscillatory motion rheological analysis.

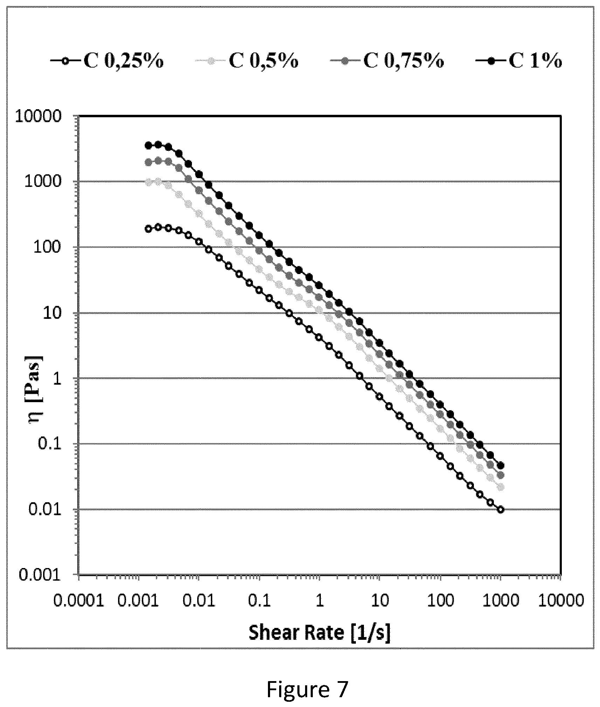

The CSR (Controlled Shear Rate) analysis simulates the application of the product on the skin and returns a flow curve which allows to study the viscosity trend as a function of the applied deformation rate. From the graph shown in figure 7 it is evident that the increase in the concentration of gum entails an increase in the viscosity of the hydrogels: at high concentrations networks and molecular associations are formed characterized by a high level of interaction. The rheological behaviour is pseudoplastic, characterized by an initial plateau in which the viscosity remains constant, which then decreases as the energy imparted increases. As the shear rate increases the network is disrupted, the molecules align in the direction of the flow, reducing size and thus viscosity.

In order to obtain more complete information on the structural features of the formulation, oscillatory motion analyses were performed: Amplitude Sweep (AS) and Frequency Sweep (FS).

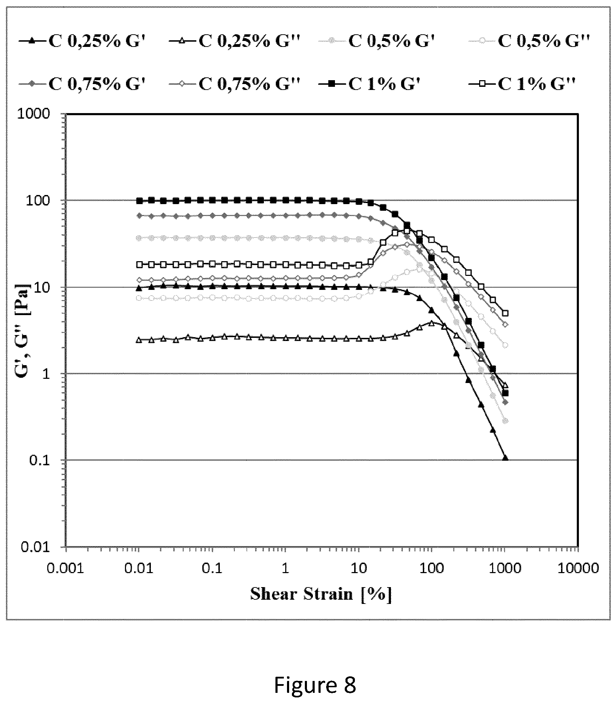

During the Amplitude Sweep, the oscillation frequency of the plate is varied, keeping the frequency instead constant. With this analysis, it is possible to identify the "linear viscoelasticity region" (LVER), i.e. the deformation range within which the sample is not subject to irreversible changes. For the hydrogels analysed, the LVER is quite large, the elastic module G' begins to decrease for shear strain values comprised between 10% and 30%, and meets the viscous module G" at the critical strain point: this indicates the breaking point of the system. Figure 8 shows that as the concentration of polysaccharide increases, the value of the modules increases, while the value of the critical strain slightly decreases: for high concentrations, the system is enriched in elastic component, it is more fragile and therefore it breaks first.

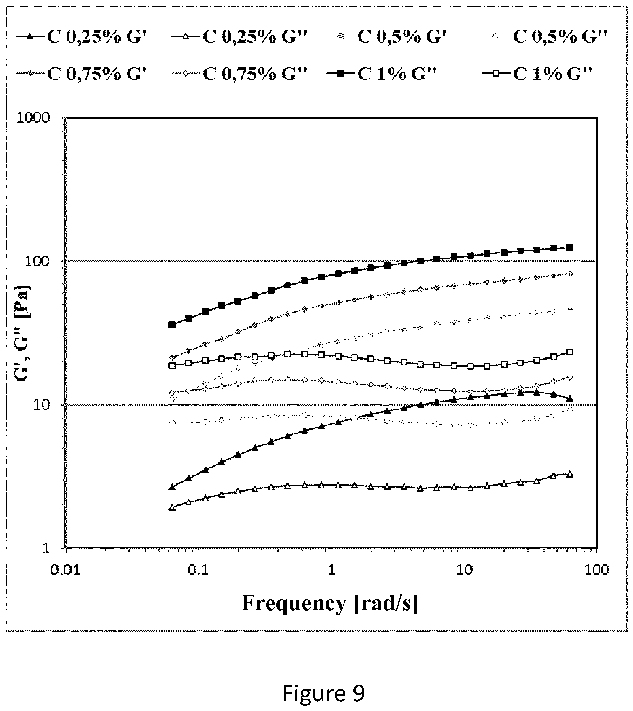

The Sweep Frequency is conducted by keeping the oscillation amplitude constant and varying the frequency. The oscillation amplitude value is taken within the LVER identified by the AS and allows an analysis to be performed which is capable of describing the microstructure of the system. For hydrogels containing *Sphingomonas* ferment extract it can be seen that, in the range of frequencies investigated, the elastic components always dominate on the viscous components and as the concentration of polymer increases, the structuring of the system also increases (Figure 9). Since the modules are comprised in the first two decades and are characterized by a trend scarcely dependent on the frequency, a weak-gel can be defined.

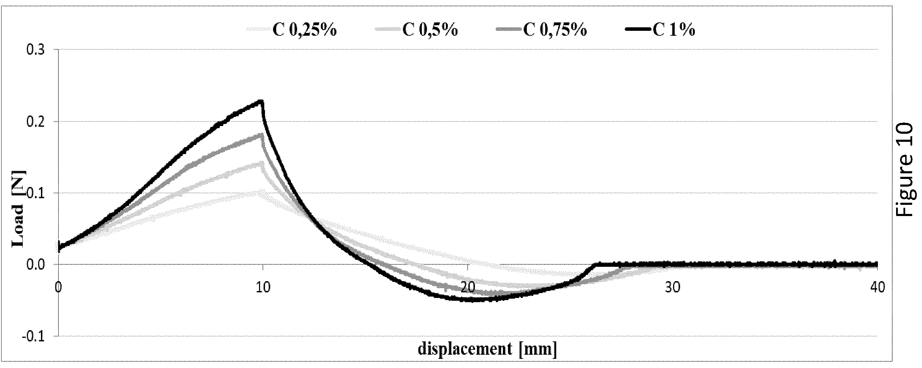

The hydrogels were finally characterized in the Texture Analyzer and the recorded parameters were compared in the graph shown in figure 10. As the polymer concentration increased, there was an increase in Adhesiveness and Cohesiveness, while the Stringiness decreased. In fact, at higher concentrations, more dense and structured polymer networks are formed in which the water molecules are effectively trapped; therefore, the samples are more consistent and stickier than those at lower gum concentrations.

Following its characterization, the gum was used for the formulation of emulsified systems containing ZINC OXIDE (A) and Zea mays starch, Polyvinyl alcohol, Glycerin

### 4.3.3 SYSTEMS AC

These systems were set up keeping the oil phase O at 40% and the concentration of *Sphingomonas* ferment extract (C) at 0.1%, the behaviour of the ZINC OXIDE concentration (A) was studied.
^{©}

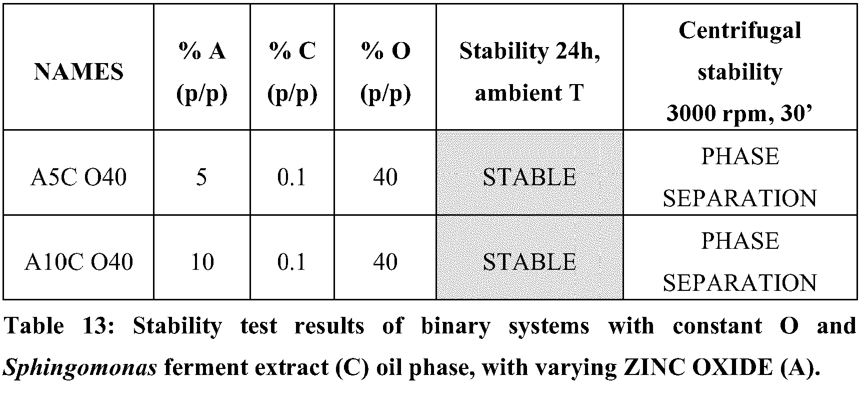

The stability of the samples did not improve with respect to the previously presented samples, indeed, in the centrifuge tests the binary systems AC report more evident separations with respect to the formulations containing only ZINC OXIDE. Probably, *Sphingomonas* ferment extract acts by viscosifying the water phase and at the same time occupying the parts of the interfaces necessary for zinc, which then precipitates more easily.

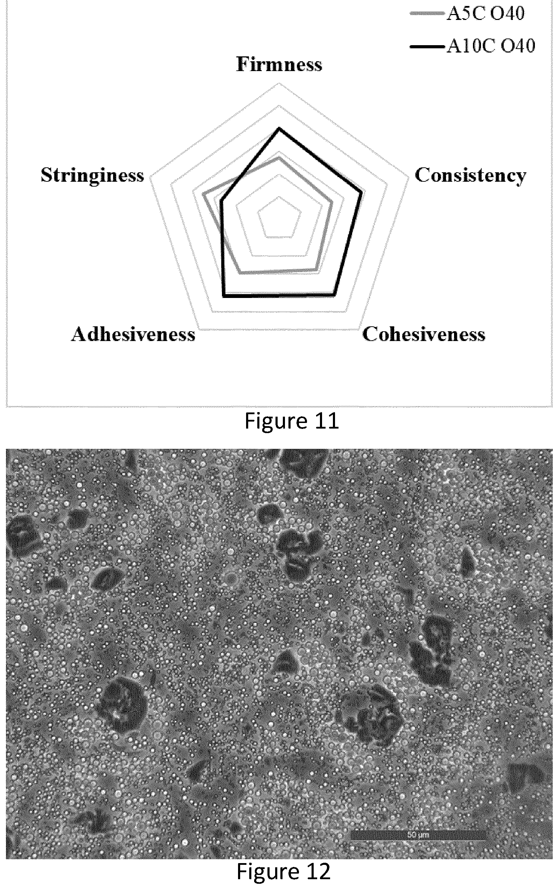

From the texture analysis it can be seen that, as the amount of ZINC OXIDE (A) increases, there is an increase in all the texture parameters except for Stringiness (Figure 11). As the solid phase increases, the body of the system increases, which seems more like a paste than an emulsion.

### 4.3.4 SYSTEMS BC

Initially, keeping the oil phase O constant at 40% and the concentration of *Sphingomonas* ferment extract (C) at 0.1 %, the varying behaviour of the concentration of Zea mays starch, Polyvinyl alcohol, Glycerin(B) was studied, as shown in table 14

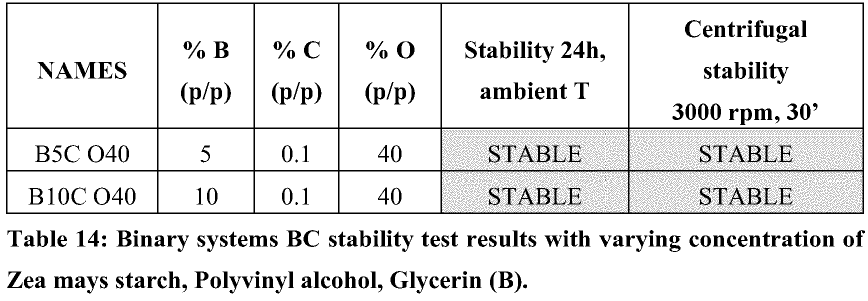

The achievement of a stable system is also confirmed by the morphological analysis of the emulsion B5C 040, performed under optical microscope with 40x magnification (Figure 12). In the image it is possible to recognize the starch granules and the presence of the polymer among the dispersed phase droplets which, unlike the previous acquisitions, are much smaller and dimensionally more homogeneous.

As the samples did not show instability phenomena during the test in the centrifuge at 3000 rpm for 30', it was thought to reduce the concentration of oils present in B5C O40, the most pleasant sensory formulation, to limit the greasiness
released at the moment of application to the skin and the results are described in Table 15.

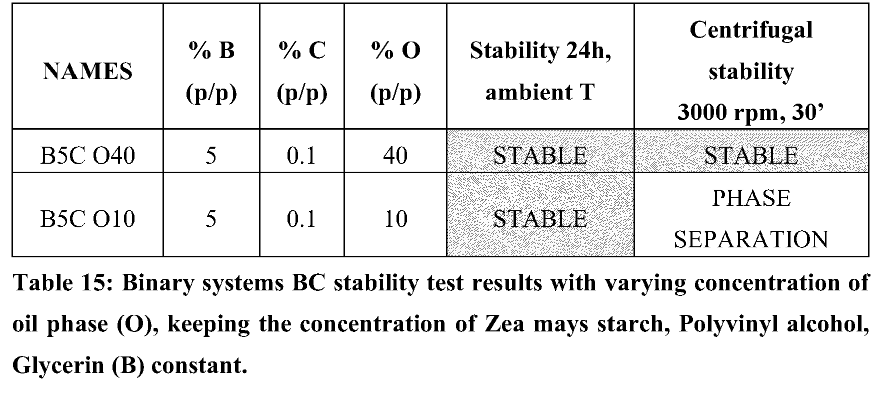

Therefore, reducing the oil phase, it is not possible to produce a system stable to the centrifuge test performed at 3000 rpm for 30 minutes: increasing the amount of water in the system, with the same gum, the system is less viscous and therefore more prone to phase separation.

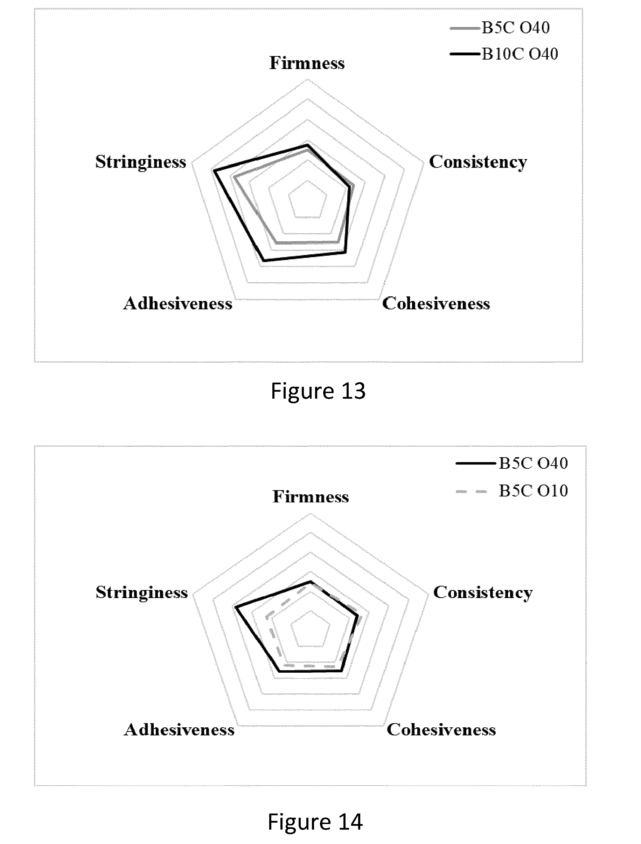

The Texture Analysis confirmed the behaviours observed previously in the type AB binary systems: even in the presence of *Sphingomonas* ferment extract, as the solid phase increases, in this case consisting of Zea mays starch, Polyvinyl alcohol, Glycerin, Adhesiveness and Stringiness increase, which describe a very sticky system, and therefore unpleasant once applied to the skin (Figure 13). On the other hand, when the oil phase decreases, the behaviour is reversed and there is a decrease in the texture parameters: in fact, the emulsion B5C O10, despite the presence of the gum, is much more fluid than the previous ones (Figure 14).

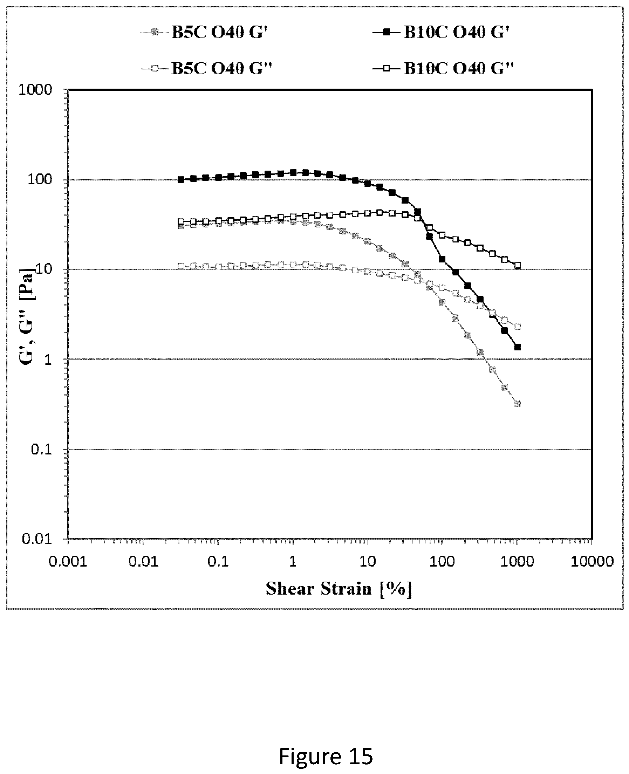

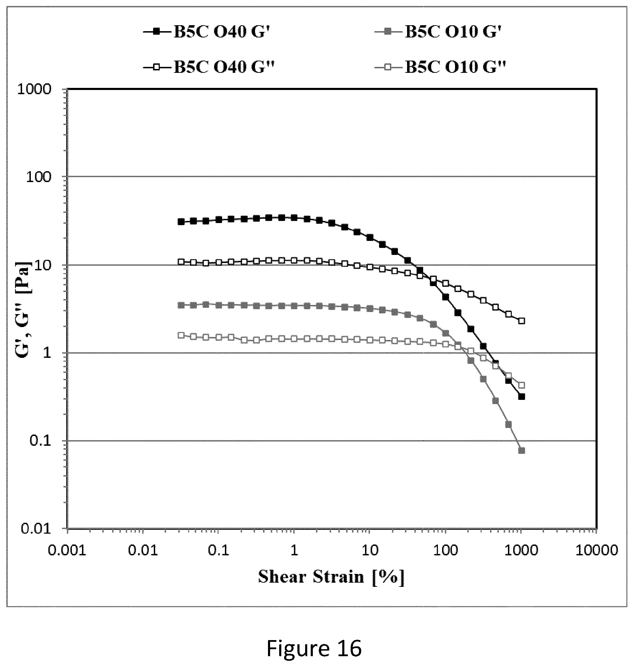

The inclusion of *Sphingomonas* ferment extract in the formula allowed to set up systems with greater homogeneity and this made it possible to perform the rheological analyses. From the Amplitude Sweep oscillatory motion analysis it is possible to see how as the concentration of starch increases there is an increase in the value of the elastic and viscous modules, while the linear range of viscoelasticity and the critical strain point are superimposable (Figure 15). Decreasing the oil phase instead results in a decrease in the value of the modules and an increase in the extent of the region in which the system does not undergo irreversible changes (Figure 16). Reducing the concentration of the oils increases the amount of water present in the system, allowing the *Sphingomonas* ferment extract to effectively bind a greater number of water molecules within its double helix; at the same time, at the same gum concentration, there is no increase in the viscosity of the system, which is therefore less elastic and more resistant to applied stress.

### 4.4. Systems emulsified by ternary powder associations

Given the positive impact of the addition of *Sphingomonas* ferment extract *gum,* emulsifying systems containing ternary associations of ZINC OXIDE (A), Zea mays starch, Polyvinyl alcohol, Glycerin (B) and *Sphingomonas* ferment extract (C), which from this moment on will be called ABC, were set up and studied in this section.

During the study, Zinc oxide and Zea mays starch, Polyvinyl alcohol, Glycerin are present in quantitative ratios 1:1.

Initially, as reported in Table 16, emulsions with constant solid and oil phase were formulated, 10% and 40% respectively, and the response of the system to increasing gum concentration was observed.

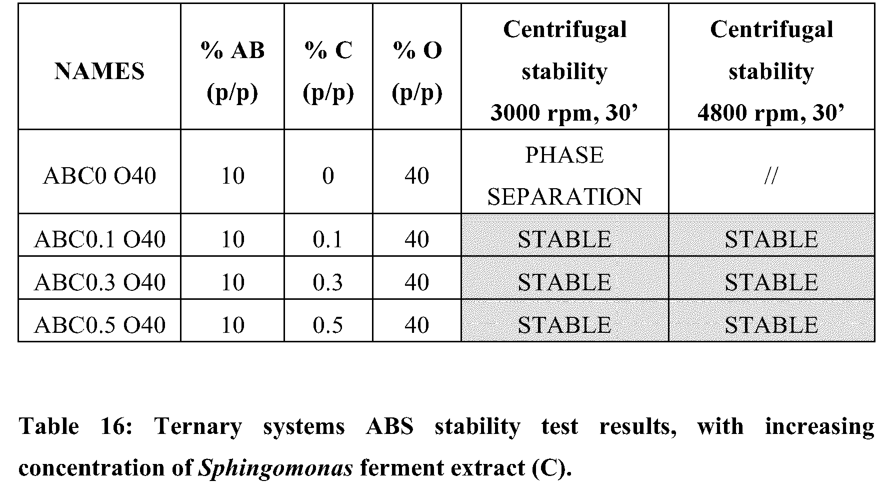

Unlike the systems previously analysed, the formulation of ternary systems allowed the preparation of stable samples in centrifuges, unchanged even after the test conducted at 4800 rpm, for 30 minutes.

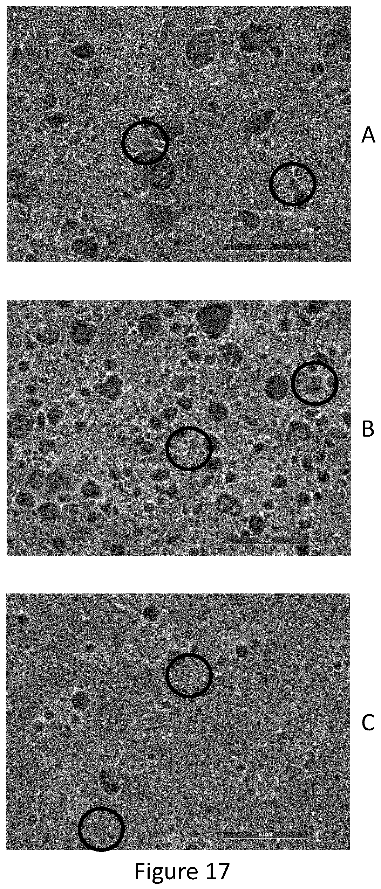

The morphological analysis under electron microscope made it possible to investigate the structure of the prepared systems: they are all characterized by a dense dispersed phase composed of very small particles, characteristic of a well-stabilized system (Figure 17). Furthermore, it can be noted that the increase in the concentration of the gum results in an increase in the visible aggregates of zinc oxide, which under the light microscope appear to be a red-brown colouration.

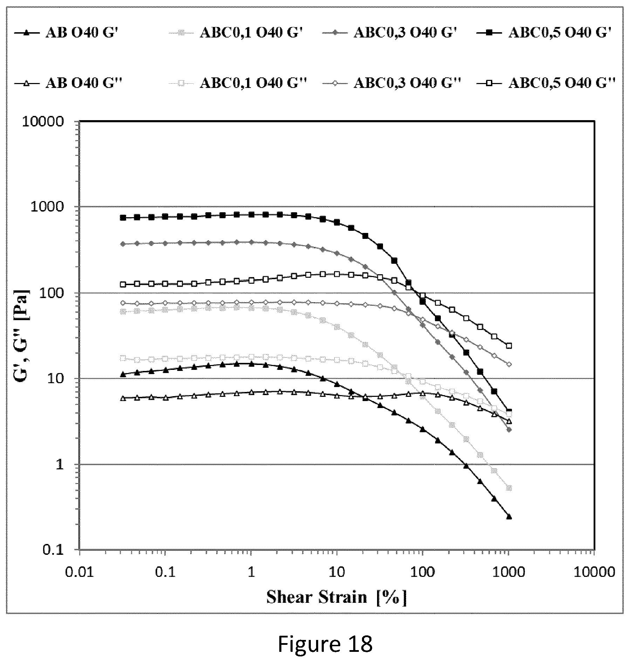

With the Amplitude Sweep analysis in oscillatory motion, conducted at constant frequency and varying the amplitude of the applied deformation, it is possible to see how the presence of *Sphingomonas* ferment extract entails an increase in the range of linear viscoelasticity consistent with the increase in the concentration of polymer used (Figure 18). As the gum concentration increases, the value of the modules increases and the critical strain point moves to greater amplitudes, a sign of greater stabilizing capacity: the amount of the elastic component present in the system increases, which is more stable and therefore more difficult to deform. Such differences are even more evident than the reference AB 10 O40, where the gum is not present.

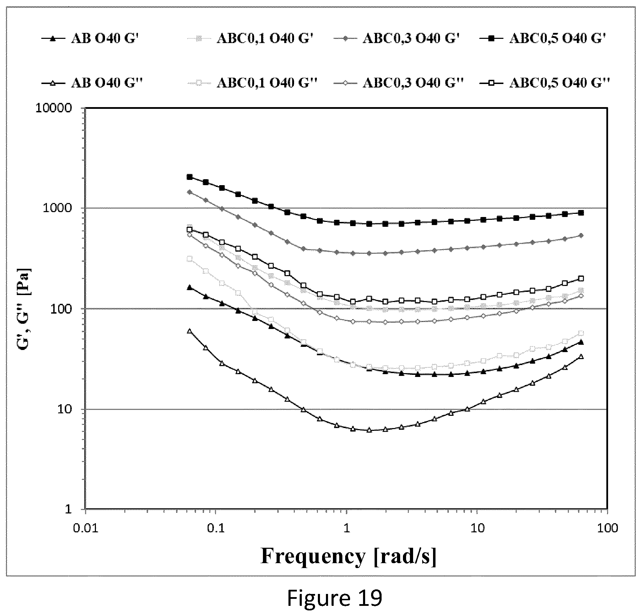

The Frequency Sweep is instead conducted by varying the oscillation frequencies and using constant shear strain values comprised in the LVER. This analysis allows the study of structural features, because the amplitude of oscillation chosen involves reversible changes in the system. In figure 19 it can be seen that, as the frequency varies, the elastic module G' is always greater than the viscous module G": the elastic components dominate over the viscous ones and therefore, all the energy imparted to the emulsion can be effectively dissipated.

Given the values of the modules, comprised in the second and third decade, it can be stated that the system is structurally considered a weak gel: it is therefore stabilized by physical interactions or low binding energy such as Van der Waals interactions and hydrogen bonds; this type of interactions confers good spreadability features to the system.

In order to obtain a less greasy and more pleasant system on the skin, the effects due to the variation of the concentration of *Sphingomonas* ferment extract in an emulsified system containing 10% total solid phase and 10% total oil phase were studied, as reported in table 17.

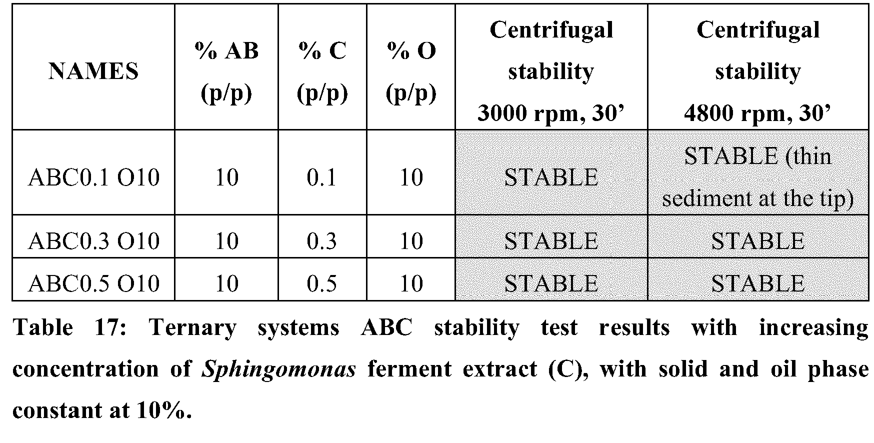

Also in this case, the introduction of *Sphingomonas* ferment extract surprisingly allowed stability to be achieved even under the high stress conditions imposed by the centrifuge test, conducted at 4800 rpm for 30 minutes. The samples were subsequently characterized by rheological analyses in oscillatory motion.

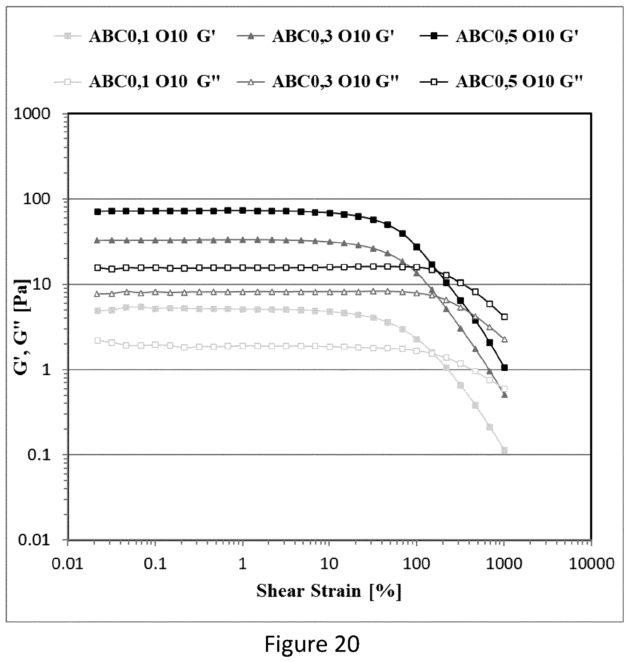

From the Amplitude Sweep it is possible to notice that the modules are located between the first and second decade; their numerical value increases with the increase of the gum concentration, without however bringing significant variations in the LVER and in the critical strain value, which indicates the point where the structure of the sample breaks and begins to flow (Figure 20).

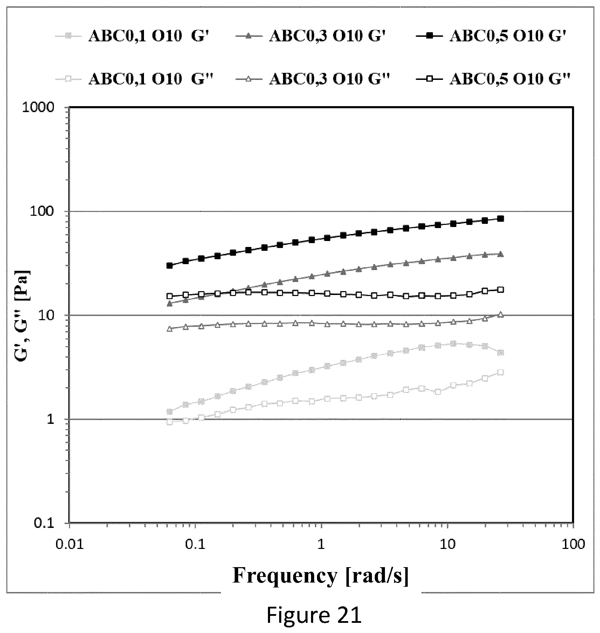

The Frequency sweep, carried out under reversible deformation conditions for the system, confirms the behaviour seen for the systems ABC O40. The system has an elastic behaviour since G' is always greater than G'', and the trend of the modules is independent of the frequency, so the structure is that of a weak-gel. (Fig.21)

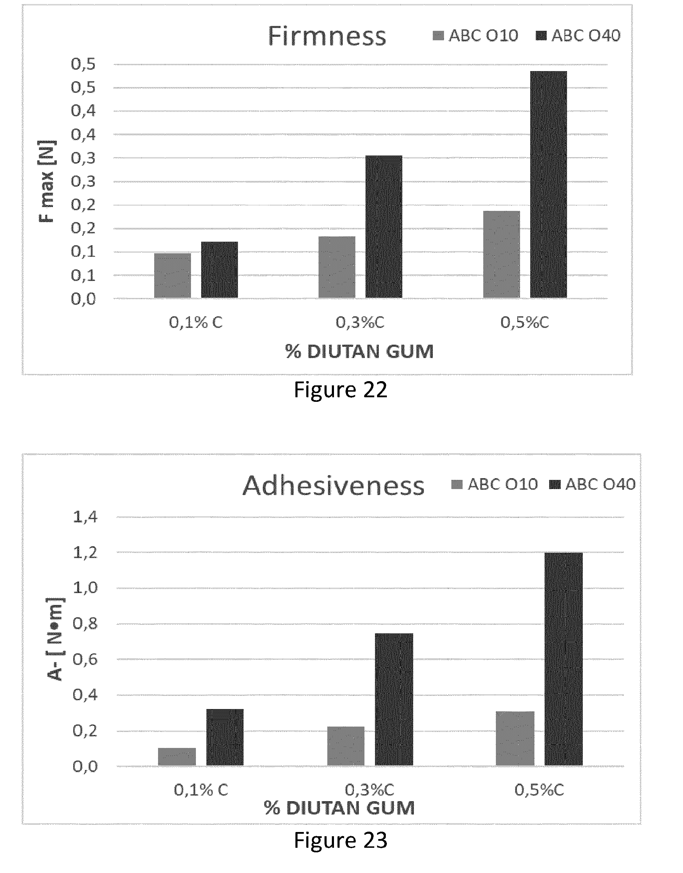

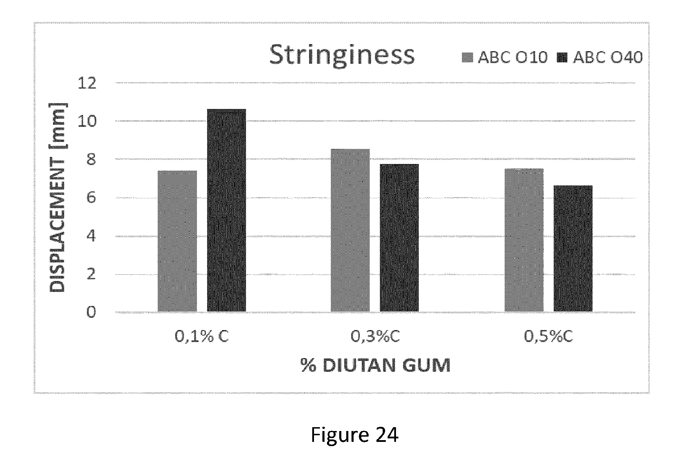

In the histograms shown in figure 22-24 it can be seen that for both sets of formulations, the increase in the gum concentration leads to an increase in the parameters of Firmness, Adhesiveness, while the Stringiness decreases. However, the increase is always much greater in the series ABC O40 with respect to ABC O10.

Since the purpose of the study is to identify a very fluid system and therefore adaptable to different uses in the cosmetic field, it was decided to investigate the behaviour of the ternary system ABC at intermediate concentrations of oil phase, keeping the phase AB constant at 10% and the gum C at 0.1% (Table 18).

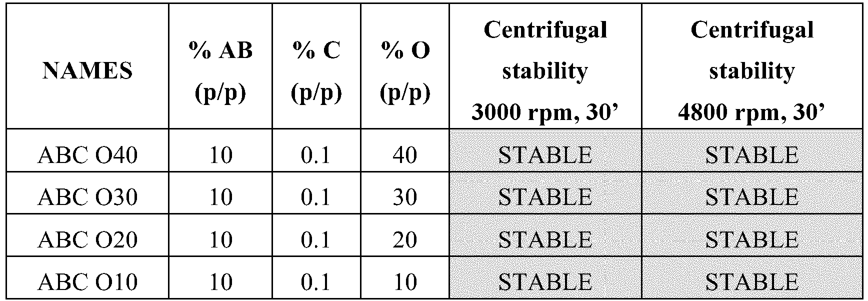

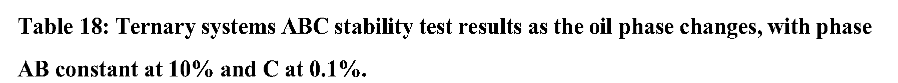

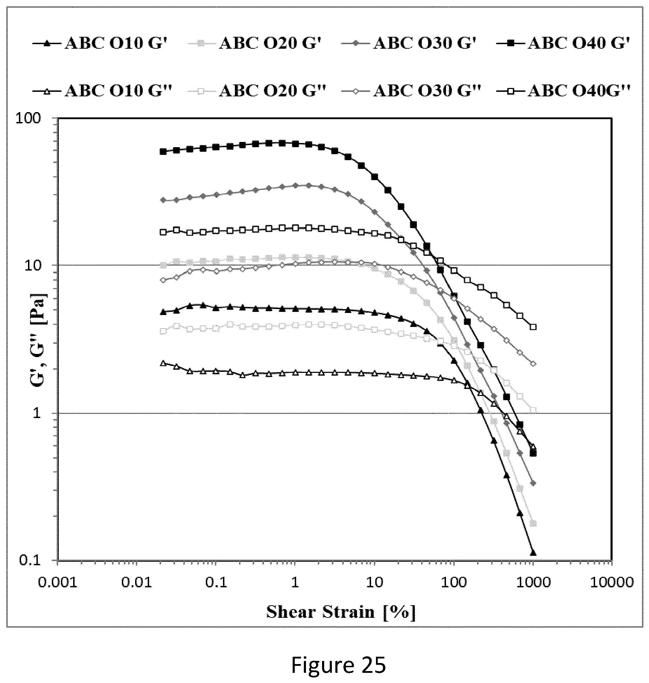

In the analysis in oscillatory motion with variable deformation amplitude (Amplitude Sweep), it can be seen that the value of the modules decreases with the decrease of the oil concentration (Figure 25). Furthermore, the increase in water phase resulting from the reduction of oils allows the *Sphingomonas* ferment extract to sequester a greater number of water molecules, making the system more resistant, moving the critical strain values to higher percentage values and extending the range of linear viscoelasticity.

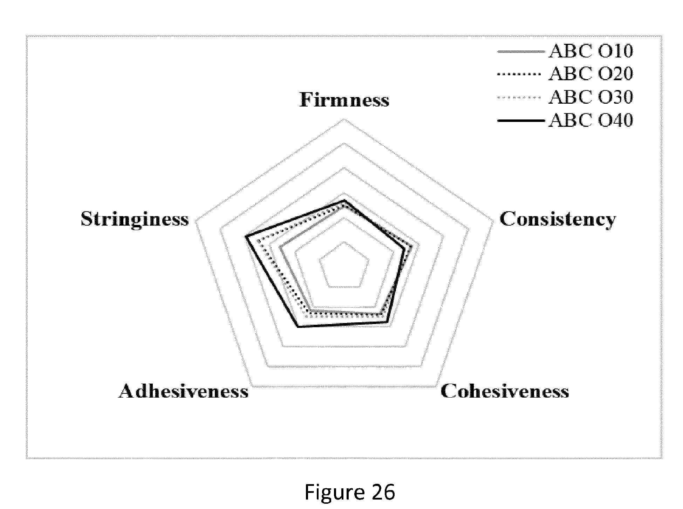

In the relative radar graph of texture parameters it can be noted that the formulations ABC O30 and ABC O20, have Firmness, Consistency and Cohesiveness values almost identical to ABC O10; their Stringiness and Adhesiveness value is instead higher than the formulation with 10% oil (Figure 26).

Among all the samples prepared, ABC O10 is the candidate which has the best features to be adapted for cosmetic use: it is capable of reconciling a satisfactory stability profile with sensory pleasantness during and after application. The use of non-high concentrations of maize starch and zinc oxide limits the sticky after-feel and white effect on the skin; *Sphingomonas* ferment extract helps to obtain a homogeneous system, easily spreadable and with a silky finish; the reduction of the oil phase concentration to 10% reduces the greasiness typical of other samples.

In the final step of this section, an attempt was made to optimize the system by reducing the aliquot of phase AB, as shown in table 19. This served to investigate the minimum concentration value of ZINC OXIDE and Zea mays starch, Polyvinyl alcohol, Glycerin necessary to confer stability after the centrifuge test and to study the differences found by the physical-mechanical characterization in rheology and texture.

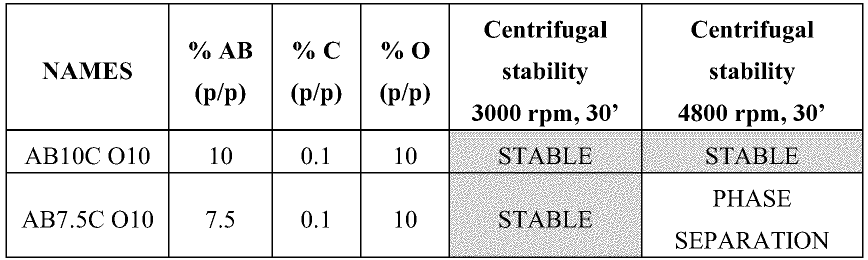

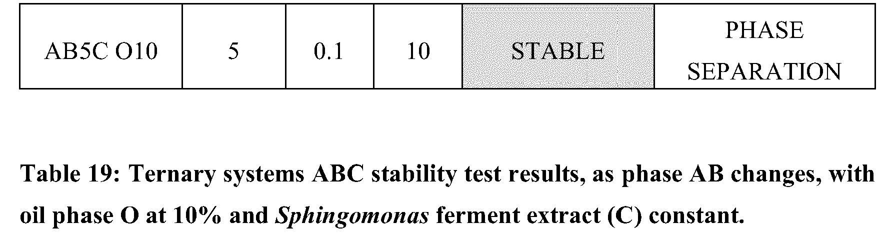

It is clear that, with the decrease in phase AB, a loss of stability is noted following the centrifuge tests conducted at 4800 rpm for 30 minutes. In fact, the systems have a significant sediment of zinc which cannot be effectively suspended in the system. The decrease in the phase AB involves a decrease in the amount of starch present in the formula: it has a hydrophilic portion capable of swelling in water, which therefore contributes to the gelification of the water phase. By reducing the starch, the amount of free water which disadvantages the adsorption of zinc at the interface is increased as already noted in the set-up of systems emulsified by a single powder. In addition, the quantitative reduction of the phase AB influences the thickness of the mechanical barrier which is created around the dispersed phase droplets: the droplets are inclined to join to try to be screened more efficiently, and this results in a reduction of the available interface area for the zinc, which, being in excess, precipitates.

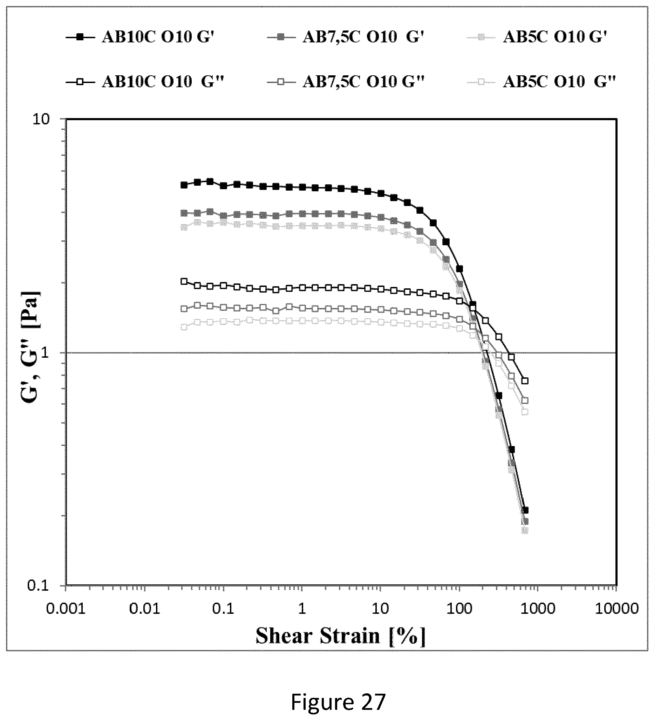

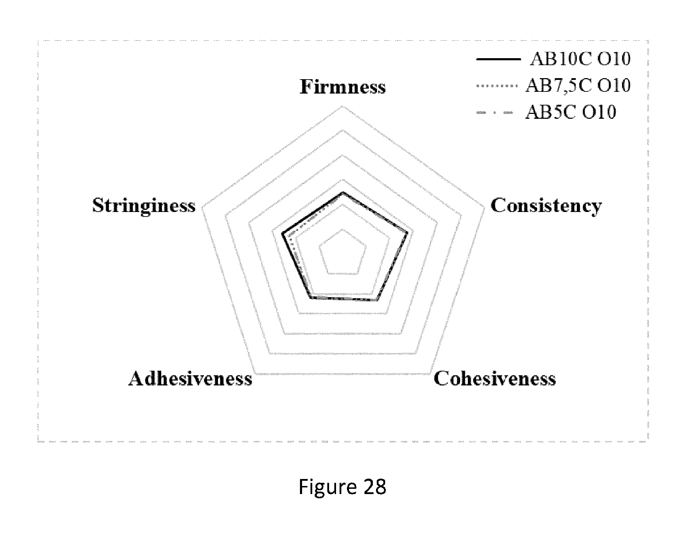

In addition to not benefiting the stability of the system, the reduction of the phase AB does not lead to improvements either from the rheological point of view (Figure 27) or to the texture parameters (Figure 28).

### 5. Conclusions

The results of this experimental study confirmed that the correct balance between the components of a system emulsified by powders is essential to obtain a stable, sensorypleasing product which is therefore potentially adaptable to the demands imposed by the cosmetic industry.

In this regard, stability tests and morphological analyses proved to be fundamental instrumental techniques to investigate the stress resistance profile of the system. On the other hand, rheology and texture analyses were useful to describe the structural features of the samples and predict their application performance.

The initial screening allowed to test the emulsifying capacity of the selected raw materials: ZINC OXIDE and Zea mays starch, Polyvinyl alcohol, Glycerin were the only ones to form stable emulsified systems in the twenty-four hours following formulation, underlining how the partial wettability for both phases of the emulsion is necessary to allow the solid phase particles to remain in the interface.

Subsequently, the achievement of physical stability was studied by setting up individual systems subjected to solid and oil phase variation. The systems stable at the centrifugal test conducted at 3000 rpm are those characterized by 40% oil phase and 15% solid phase, both for ZINC OXIDE and for Zea mays starch, Polyvinyl alcohol, Glycerin. More like a paste than an emulsion, these systems are very unpleasant when applied to the skin. The high percentage of oil leaves a greasy residue after application, while the high concentrations of zinc and maize starch lead to a pronounced "white" effect and an annoying sticky after-feel, respectively.

In order to improve both the system stability and sensoriality, emulsified systems were set up by the association of ZINC OXIDE and Zea mays starch, Polyvinyl alcohol, Glycerin, in a 1:1 ratio. Since the desired results were not achieved, further binary systems were set up, associating each of the two powders used with *Sphingomonas* ferment extract, a polysaccharide from bacterial fermentation with a suspending and viscosifying action. The systems containing zinc and gum reported the same instability as the systems emulsified by single powder, while the combination of gum and starch allowed the production of samples stable to centrifuge tests only for high percentages of oil phase (40%).

Finally, emulsified systems containing ternary associations of ZINC OXIDE and Zea mays starch, Polyvinyl alcohol, Glycerin, present in a constant 1:1 ratio and Sphingomonas ferment extract were formulated and studied. The insertion of the gum helped system stabilization even at 4800 rpm and the suspension of the components; therefore, the finished product is more homogeneous with respect to the previous formulations, and was also improved in sensoriality.

The rheological and texture analyses performed demonstrated the solid-like behaviour of the systems, which from a structural perspective are weak-gels stabilized by physical interactions or soft chemical interactions. ABC O10 was identified as the best candidate among all the samples formulated: the fluid consistency and good flow capabilities, combined with stability and balanced sensory features make it easily adaptable to use in the cosmetic field.

## Claims

1. Stable oil-in-water emulsion free of surfactants, comprising:
(i) zinc oxide powder;
(ii) a powder mixture comprising or consisting of maize starch;
(iii) a powder comprising or consisting of polysaccharide from bacterial fermentation;
(iv) an oil phase;
(v) water;
wherein
(I) the total concentration of (i)+(ii) is comprised between 3% and 12% by weight on total weight of said oil-in-water emulsion;
(II) the concentration of (iii) is comprised between 0.05% and 0.7% by weight on the total weight of said oil-in-water emulsion;
(III) the concentration of the oil phase (iv) is comprised between 5% and 50% by weight on the total weight of said emulsion;
(IV) the weight ratio of (i)/(ii) is comprised between 1:5 and 5:1;
(V) (ii) is a maize starch mixture containing between 50 and 75% of said starch, between 25 and 50% by weight of polyvinyl alcohol and between 5 and 10% by weight of glycerine on the total weight of the mixture;
(VI) (iii) is an extract fermented from *Sphingomonas* and consists of mixtures of polysaccharides with average molecular weight comprised between 2.85 and 5.20 million Da at low charge density in which the repeating unit in the chain consists of 4 sugars, of which one is functionalized with a carboxylate group, while the side chain consists of repeating units with two neutral sugars.

2. Oil-in-water emulsion according to claim 1, wherein the total concentration of (i)+(ii) is comprised between 5% and 10% weight on the total weight of said oil-in-water emulsion.

3. Emulsion according to claim 1 or 2, wherein the concentration of (iii) is comprised between 0.1- and 0.5% by weight on total weight of said oil-in-water emulsion.

4. Emulsion according to any one of claims 1-3, wherein the concentration of the oil phase is comprised between 10% and 40% by weight on the total weight of said oil-in-water emulsion.

5. Oil-in-water emulsion according to any one of claims 1-4, wherein the weight ratio (i)/(ii) is comprised between 2:1 and 1:2.

6. Oil-in-water emulsion according to claim 5, wherein said weight ratio (i)/(ii) is 1:1.

7. Cosmetic formulation comprising or consisting of the oil-in-water emulsion according to any one of claims 1-6.

8. Cosmetic formulation according to claim 7, for use as topical skin treatment.

## Patentansprüche

1. Stabile Öl-in-Wasser-Emulsion frei von Tensiden, umfassend:
(i) Zinkoxidpulver;
(ii) ein Pulvergemisch, umfassend oder bestehend aus Maisstärke;
(iii) ein Pulver, umfassend oder bestehend aus Polysaccharid aus bakterieller Fermentation;
(iv) eine Ölphase;
(v) Wasser;
wobei
(I) die Gesamtkonzentration von (i)+(ii) zwischen 3 und 12 Gewichts-%, bezogen auf das Gesamtgewicht der Öl-in-Wasser-Emulsion, liegt;
(II) die Konzentration von (iii) zwischen 0,05 und 0,7 Gewichts-%, bezogen auf das Gesamtgewicht der Öl-in-Wasser-Emulsion, liegt;
(III) die Konzentration der Ölphase (iv) zwischen 5 und 50 Gewichts-%, bezogen auf das Gesamtgewicht der Emulsion, liegt;
(IV) das Gewichtsverhältnis von (i)/(ii) zwischen 1:5 und 5:1 liegt;
(V) (ii) ein Maisstärkegemisch ist, die zwischen 50 und 75 % der Stärke, zwischen 25 und 50 Gewichts-% Polyvinylalkohol und zwischen 5 und 10 Gewichts-% Glycerin, bezogen auf das Gesamtgewicht des Gemischs, enthält;
(VI) (iii) ein aus *Sphingomonas* fermentierter Extrakt ist und aus Gemischen von Polysacchariden mit einem durchschnittlichen Molekulargewicht zwischen 2,85 und 5,20 Millionen Da bei niedriger Ladungsdichte besteht, wobei die sich wiederholende Einheit in der Kette aus 4 Zuckern besteht, wovon einer mit einer Carboxylatgruppe funktionalisiert ist, während die Seitenkette aus Wiederholungseinheiten mit zwei neutralen Zuckern besteht.

2. Öl-in-Wasser-Emulsion nach Anspruch 1, wobei die Gesamtkonzentration von (i)+(ii) zwischen 5 und 10 Gewichts-%, bezogen auf das Gesamtgewicht der Öl-in-Wasser-Emulsion, liegt.

3. Emulsion nach Anspruch 1 oder 2, wobei die Konzentration von (iii) zwischen 0,1 und 0,5 Gewichts-%, bezogen auf das Gesamtgewicht der Öl-in-Wasser-Emulsion, liegt.

4. Emulsion nach einem der Ansprüche 1-3, wobei die Konzentration der Ölphase zwischen 10 und 40 Gewichts-%, bezogen auf das Gesamtgewicht der Öl-in-Wasser-Emulsion, liegt.

5. Öl-in-Wasser-Emulsion nach einem der Ansprüche 1-4, wobei das Gewichtsverhältnis (i)/(ii) zwischen 2:1 und 1:2 liegt.

6. Öl-in-Wasser-Emulsion nach Anspruch 5, wobei das Gewichtsverhältnis (i)/(ii) 1:1 ist.

7. Kosmetische Formulierung, umfassend oder bestehend aus der Öl-in-Wasser-Emulsion nach einem der Ansprüche 1-6.

8. Kosmetische Formulierung nach Anspruch 7 zur Verwendung als topische Hautbehandlung.

## Revendications

1. Émulsion huile-dans-eau stable exempte de tensioactifs, comprenant :
(i) de la poudre d'oxyde de zinc ;
(ii) un mélange de poudre comprenant ou consistant en de l'amidon de maïs ;
(iii) une poudre comprenant ou consistant en un polysaccharide issu de la fermentation bactérienne ;
(iv) une phase huileuse ;
(v) de l'eau ;
dans laquelle
(I) la concentration totale de (i)+(ii) est comprise entre 3 % et 12 % en poids par rapport au poids total de ladite émulsion huile-dans-eau ;
(II) la concentration de (iii) est comprise entre 0,05 % et 0,7 % en poids par rapport au poids total de ladite émulsion huile-dans-eau ;
(III) la concentration de la phase huileuse (iv) est comprise entre 5 % et 50 % en poids par rapport au poids total de ladite émulsion ;
(IV) le rapport en poids de (i)/(ii) est compris entre 1:5 et 5:1 ;
(V) (ii) est un mélange d'amidon de maïs contenant entre 50 et 75 % dudit amidon, entre 25 et 50 % en poids d'alcool polyvinylique et entre 5 et 10 % en poids de glycérine par rapport au poids total du mélange ;
(VI) (iii) est un extrait fermenté de *Sphingomonas* et est constitué de mélanges de polysaccharides de poids moléculaire moyen compris entre 2,85 et 5,20 millions Da à faible densité de charge dans lesquels l'unité répétitive dans la chaîne est constituée de 4 sucres, dont l'un est fonctionnalisé avec un groupe carboxylate, tandis que la chaîne latérale est constituée d'unités répétitives avec deux sucres neutres.

2. Émulsion huile-dans-eau selon la revendication 1, dans laquelle la concentration totale de (i)+(ii) est comprise entre 5 % et 10 % en poids par rapport au poids total de ladite émulsion huile-dans-eau.

3. Émulsion selon la revendication 1 ou 2, dans laquelle la concentration de (iii) est comprise entre 0,1 et 0,5 % en poids par rapport au poids total de ladite émulsion huile-dans-eau.

4. Émulsion selon l'une quelconque des revendications 1 à 3, dans laquelle la concentration de la phase huileuse est comprise entre 10 % et 40 % en poids par rapport au poids total de ladite émulsion huile-dans-eau.

5. Émulsion huile-dans-eau selon l'une quelconque des revendications 1 à 4, dans laquelle le rapport en poids (i)/(ii) est compris entre 2:1 et 1:2.

6. Émulsion huile-dans-eau selon la revendication 5, dans laquelle ledit rapport en poids (i)/(ii) est de 1:1.

7. Formulation cosmétique comprenant ou consistant en l'émulsion huile-dans-eau selon l'une quelconque des revendications 1 à 6.

8. Formulation cosmétique selon la revendication 7, destinée à être utilisée comme traitement cutané topique.
